# EUROPÄISCHE PATENTSCHRIFT

(11) **EP 3 612 671 B1**
(45) Veröffentlichungstag und Bekanntmachung des Hinweises auf die Patenterteilung: **12.10.2022**
(21) Anmeldenummer: 18719561.5
(22) Anmeldetag: 23.04.2018
(51) Int. Cl.: D04B 1/12

(54) **FORMGESTRICK UND VERWENDUNG EINES FORMGESTRICKS**
SHAPE KNIT FABRIC AND USE OF A SHAPE KNIT FABRIC
TRICOT DE FORME ET UTILISATION D'UN TRICOT DE FORME

(30) Priorität: 21.04.2017 DE 102017108550
(43) Veröffentlichungstag der Anmeldung: 26.02.2020
(73) Patentinhaber: Pilz GmbH & Co. KG, 73760 Ostfildern (DE)
(72) Erfinder: HORTER, Hansjürgen, 73760 Ostfildern (DE); GÖNNER, Karl, 73760 Ostfildern (DE); RIEDER, Oswald, 73770 Denkendorf (DE); RÖDER, Uwe, 73770 Denkendorf (DE); PILZ, Thomas, 73760 Ostfildern (DE)
(74) Vertreter: Manske, Jörg
(86) Internationale Anmeldenummer: PCT/EP2018/060367
(87) Internationale Veröffentlichungsnummer: WO 2018/193135

(56) Entgegenhaltungen:
- WO-A1-2005/091319
- WO-A1-2013/120624
- WO-A2-99/64657

## Beschreibung

Die vorliegende Erfindung betrifft ein Formgestrick nach dem Oberbegriff des Anspruchs 1 sowie eine Verwendung eines Formgestricks.

### Definition:

Unter dem Begriff "Formgestrick" sollen im Rahmen dieser Anmeldung Gestricke verstanden werden, die eine zweidimensionale oder eine dreidimensionale Struktur aufweisen und zumindest abschnittsweise stricktechnisch hergestellt sind.

### Stand der Technik

Elektrisch leitende Textilstrukturen, insbesondere sensorische Gewebe, sind aus dem Stand der Technik in unterschiedlichen Ausführungsformen bekannt. Diese können beispielsweise als Sensoren in unterschiedlichen technischen Applikationen eingesetzt werden.

Aus der US 4,795,998 A ist ein sensorisches Gewebe mit einer Gewebelage bekannt, wobei sich elektrisch leitende Fäden der Gewebelage an bestimmten Kreuzungsstellen kreuzen. In Abhängigkeit von einer auf das sensorische Gewebe einwirkenden äußeren Kraft verändert sich der Übergangswiderstand zwischen den an den Kreuzungsstellen aneinander anliegenden Fäden. Auf diese Weise ist es möglich, eine Kraft, die an einer der Kreuzungsstellen der elektrisch leitenden Fäden auf das Gewebe einwirkt, durch die Bestimmung der Änderung eines elektrischen Übergangswiderstands mittels einer geeigneten Auswerteschaltung zu erfassen.

Ein für sensorische Aufgaben verwendbares Gestrick unterscheidet sich in seinen Eigenschaften und durch eine vollkommen unterschiedliche Art der Herstellung deutlich von einem Gewebe, da es Maschen bildet. Ein derartiges Gestrick ist zum Beispiel aus der EP 1 997 952 A2 bekannt und soll nachfolgend unter Bezugnahme auf Fig. 1 und 2 näher erläutert werden.

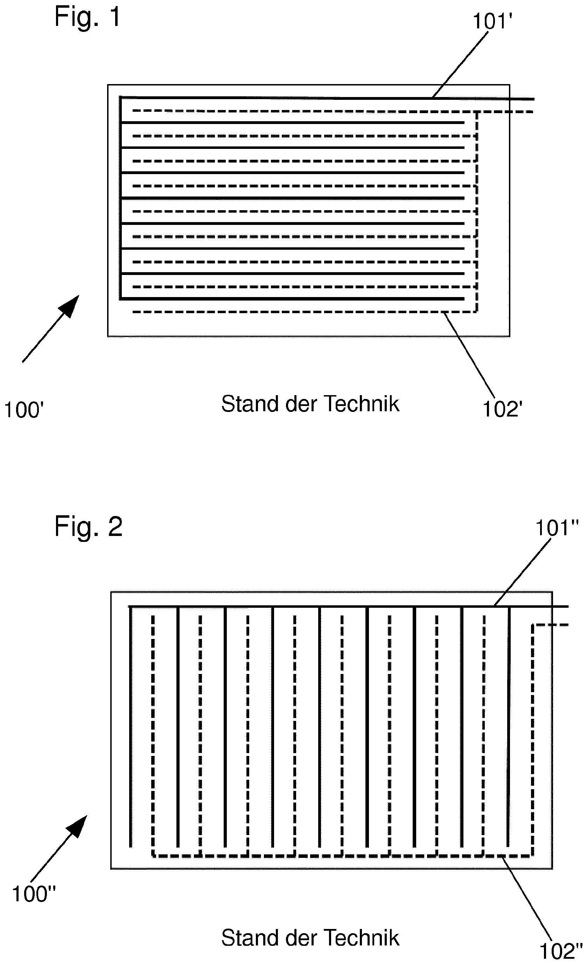

Fig. 1 zeigt ein einlagiges Intarsia-Gestrick 100' mit elektrisch leitfähigen und elektrisch nicht leitfähigen Bereichen. In das Intarsia-Gestrick 100' sind elektrisch leitende Garne so eingestrickt, dass zwei ineinandergreifende Kammstrukturen 101', 102' gebildet werden. Die beiden Kammstrukturen 101', 102' bilden hierbei zwei ineinandergreifende, in einer ersten (waagerechten) Richtung orientierte Elektroden zur kapazitiven Flächenüberwachung. Eine erste Kammstruktur 101' wird an einen Pluspol einer Spannungsversorgungseinrichtung einer Auswerteschaltung angeschlossen. Eine zweite Kammstruktur 102' wird demgegenüber an einen Minuspol der Spannungsversorgungseinrichtung der Auswerteschaltung angeschlossen. Mit anderen Worten ist also eine der beiden Kammstrukturen 101', 102' des Gestricks 100' nach dem Anschluss an die Spannungsversorgungseinrichtung positiv und die andere der beiden Kammstrukturen 101', 102' negativ geladen. Damit kann zum Beispiel eine Annäherung an das Gestrick 100', in dem die beiden Kammstrukturen 101', 102' ausgebildet sind, kapazitiv überwacht werden. Dabei wirkt das Gestrick 100' aus technisch-funktionaler Sicht wie eine Mehrzahl offener Kondensatoren, die jeweils ein elektrisches Feld bilden. Zwischen den beiden Kammstrukturen 101', 102', die an die beiden Pole der elektrischen Spannungsversorgungseinrichtung der Auswerteschaltung angeschlossen sind, werden somit elektrische Felder gebildet. Sobald ein Objekt mit einer Dielektrizitätszahl, die größer als die Dielektrizitätszahl von Luft ist, in eines der elektrischen Felder eindringt, wird sich je nach Material dieses Objekts die Kapazität des elektrischen Feldes verändern, insbesondere vergrößern. Diese Veränderung kann mittels einer entsprechenden Auswerteeinheit der Auswerteschaltung, die an die Kammstrukturen 101', 102' angeschlossen ist, gemessen und ausgewertet werden.

Fig. 2 zeigt eine alternative Ausführungsform eines einlagigen Intarsia-Gestricks 100", bei dem die elektrisch leitfähigen Garne so eingestrickt sind, dass die beiden ineinandergreifenden Kammstrukturen 101", 102" eine Orientierung in einer zweiten (senkrechten) Richtung aufweisen.

Mittels der in der vorstehend erläuterten Weise angeordneten, jeweils eine positiv beziehungsweise negativ geladene Elektrode bildenden Kammstrukturen 101', 102', 101", 102" der Gestricke 100', 100" ist lediglich eine Überwachung der gesamten Fläche - ohne eine entsprechende Ortsauflösung - des betreffenden Intarsia-Gestricks 100', 100" möglich, da alle elektrischen Leiter einer jeden Kammstruktur 101', 102', 101", 102" elektrisch miteinander verbunden sind.

Ein Formgestrick der eingangs genannten Art, umfassend zumindest eine erste Lage, in die mehrere linienartige oder flächenartige, insbesondere streifenförmige, elektrisch leitende Strukturen aus einem elektrisch leitfähigen Garn und linienartige oder flächenartige, insbesondere streifenförmige, elektrisch nichtleitende Strukturen aus einem elektrisch nicht leitfähigen Garn derart eingestrickt sind, dass die elektrisch leitenden Strukturen elektrisch voneinander isoliert sind, wobei jede der elektrisch leitenden Strukturen individuell elektrisch kontaktierbar und an eine Auswerteschaltung anschließbar ist, ist zum Beispiel aus der WO 2013/120624 A1 bekannt.

Die Erfindung macht es sich zur Aufgabe, ein weiter verbessertes Formgestrick zur Verfügung zu stellen, das einfach und kostengünstig herstellbar ist und sich insbesondere auch für sensorische Anwendungen oder Schaltanwendungen mit Ortsauflösung eignet.

Die Lösung dieser Aufgabe liefert ein Formgestrick der eingangs genannten Art mit den Merkmalen des Anspruchs 1. Die Unteransprüche betreffen vorteilhafte Weiterbildungen der Erfindung.

Ein erfindungsgemäßes Formgestrick zeichnet sich dadurch aus, dass die elektrisch leitenden Strukturen zur elektrischen Kontaktierung punktuell mit isolierten Mikrokabeln oder isolierten leitfähigen Garnen oder Umwindegarnen, die an der Kontaktierungsstelle partiell abisoliert sind, elektrisch verbunden sind, wobei die isolierten Mikrokabel oder isolierten leitfähigen Garne oder Umwindegarne als Stehfäden ausgebildet sind, welche sich parallel zu Maschenstäbchen des Formgestricks erstrecken. Das erfindungsgemäße Formgestrick kann im Vergleich zu den aus dem Stand der Technik vorbekannten Formgestricken aufgrund der individuellen Kontaktierbarkeit der elektrisch leitenden Strukturen unter anderem auch für Sensorapplikationen sowie Schaltapplikationen mit Ortsauflösung verwendet werden kann. Intarsiagestricke werden dadurch hergestellt, dass ein Faden nicht über die gesamte Breite des Formgestricks geführt wird, sondern in einer Maschenreihe mit einem anderen Faden eines anderen Garns an einer bestimmten Position wechselt. Damit lassen sich Flächenabschnitte des Formgestricks mit unterschiedlichen Eigenschaften auf einfache Weise aneinanderbinden. Werden bei einem Formgestrick alle Maschen gleichgroß und auf allen beteiligten Nadeln in gleicher Anzahl gestrickt, kann zum Beispiel eine rechteckige ebene Gestrickfläche erhalten werden. Erfindungsgemäß ist dabei vorgesehen, dass die elektrisch leitenden Strukturen zur elektrischen Kontaktierung punktuell mit isolierten Mikrokabeln oder isolierten leitfähigen Garnen oder Umwindegarnen, die an der Kontaktierungsstelle partiell abisoliert sind, elektrisch verbunden sind, wobei die isolierten Mikrokabel oder isolierten leitfähigen Garne oder Umwindegarne als Stehfäden ausgebildet sind, welche sich parallel zu Maschenstäbchen des Formgestricks erstrecken. Die Stehfäden bilden dabei keine Maschen des Formgestricks aus.

In einer bevorzugten Ausführungsform wird vorgeschlagen, dass die elektrisch leitenden Strukturen und/oder die elektrisch nichtleitenden Strukturen der ersten Lage als Intarsiamuster oder eingeringelte Flächen ausgebildet sind.

Um eine Gestrickfläche herzustellen, die der Abwicklung einer Oberfläche eines Formkörpers entspricht, können zum Beispiel stricktechnische Methoden, insbesondere unterschiedliche Maschengrößen, das Umhängen von Maschen (zum Beispiel mittels Hilfsnadelbetten), Stricken, Fangstricken, Maschensplitten und Nichtstricken von ausgewählten Nadeln etc., angewandt werden. Daraus ergibt sich eine große Variantenvielfalt der erfindungsgemäßen Formgestricke, die durch technische Gewebe mit elektrisch leitenden und elektrisch nichtleitenden Strukturen nicht ohne weiteres oder gar nicht erhalten werden kann.

Gemäß einem Aspekt kann durch das erfindungsgemäße Formgestrick eine annäherungsempfindliche, ortsauflösende sensorische Fläche (Oberfläche) bereitgestellt werden, die insbesondere in einem Flachstrickverfahren hergestellt werden kann. Mittels einer entsprechenden Auswerteschaltung, die an die elektrisch leitenden Strukturen des Formgestricks angeschlossen ist, können bei einer Annäherung eines Objektes die Änderungen der elektrischen Felder und/oder anderer elektrischer Zustandsgrößen erfasst werden.

Ein zumindest einlagiges Formgestrick, das die in erfindungsgemäßer Weise hergestellten elektrisch leitenden und elektrisch nichtleitenden Strukturen aufweist, bietet gegenüber einem Gewebe, das elektrisch leitende und elektrisch nichtleitende Strukturen aufweist, insbesondere folgende Vorteile:
- verbesserte Dehneigenschaften, wobei diese Dehneigenschaften durch die Maschenstrukturen des Formgestricks und nicht durch die zur Herstellung des Formgestricks verwendeten Garne hervorgerufen werden,
- eine deutlich flexiblere Struktur als ein im Vergleich dazu eher steifes Gewebe,
- eine andere Art der Zugentlastung,
- eine deutlich verbesserte Drapierbarkeit, insbesondere auch auf komplex ausgestalteten zweidimensionalen oder dreidimensionalen Oberflächen,
- die Möglichkeit einer dreidimensionalen Formgestaltung des Formgestricks bereits bei dessen Herstellung.

Das einlagige Formgestrick kann insbesondere zur Bildung einer Sensoranordnung in geeigneter Weise an eine elektrische Spannungsversorgungseinrichtung einer Auswerteschaltung angeschlossen werden, so dass die einzelnen elektrisch leitenden Strukturen des Formgestricks individuell elektrisch kontaktiert werden können. Mit anderen Worten weist das einlagige Formgestrick somit partielle Elektrodenflächen auf, die durch die elektrisch leitenden Strukturen gebildet werden und individuell elektrisch kontaktiert werden können. Aufgrund der an ihnen anliegenden elektrischen Spannung können diese elektrisch leitenden Strukturen elektrische Felder zueinander ausbilden, so dass eine Annäherung von Gegenständen und/oder Personen beziehungsweise Körperteilen mittels der Auswerteeinheit der Auswerteschaltung mit Ortsauflösung erfasst werden kann. Mittels der Auswerteeinheit können somit bei einer Annäherung eines Objektes die Änderungen der elektrischen Felder zwischen den elektrisch leitenden Strukturen erfasst werden. Das einlagige Formgestrick kann zum Beispiel als kapazitiver Annäherungssensor einer Schutzeinrichtung zur Überwachung einer technischen Anlage verwendet werden.

In einer vorteilhaften Weiterbildung wird vorgeschlagen, dass das Formgestrick zumindest eine zweite Lage aufweist, die mit der ersten Lage verbunden ist. Dadurch ist es möglich, dem Formgestrick erweiterte Funktionalitäten beziehungsweise zusätzliche Eigenschaften zur Verfügung zu stellen.

In einer besonders vorteilhaften Ausführungsform kann vorgesehen sein, dass die zweite Lage eine gestrickte Lage ist, die insbesondere stricktechnisch mit der ersten Lage verbunden ist oder mit der ersten Lage vernäht ist. Insbesondere bei einer stricktechnischen Verbindung der Lagen ergeben sich erhebliche Vorteile bei der Herstellung des Formgestricks, da die erste Lage und die zweite Lage des Formgestricks in einem einzigen Strickprozess, insbesondere in einer Flachstrickvorrichtung, hergestellt werden können. Die zweite Lage kann vollständig oder in einer alternativen Ausführungsform auch nur abschnittsweise aus einem Gestrick bestehen.

In weiteren alternativen Ausführungsformen kann die zweite Lage zumindest abschnittsweise ein Gewebe und/oder ein Gewirk und/oder ein Gelege und/oder ein Vliesmaterial und/oder ein Schaummaterial und/oder eine Folie umfassen oder vollständig daraus bestehen. Die in dieser Weise ausgebildete zweite Lage kann bei der Herstellung zum Beispiel durch ein textiles Verbindungsverfahren, beispielsweise durch Vernähen, oder auch stoffschlüssig, insbesondere durch Verkleben, mit der ersten Lage verbunden werden.

Die zweite Lage kann gemäß einer vorteilhaften Ausführungsform zum Beispiel zumindest abschnittsweise aus einem elektrisch nichtleitenden Werkstoff hergestellt sein. Vorzugsweise kann die zweite Lage so ausgebildet sein, dass sie einen Berührungsschutz für die erste Lage und/oder eine elektrische Isolation für die elektrisch leitenden Strukturen der ersten Lage bildet.

In einer besonders vorteilhaften Ausführungsform besteht die Möglichkeit, dass die zweite Lage aus einem elastisch verformbaren Werkstoff hergestellt ist. Durch diese Maßnahme wird erreicht, dass die zweite Lage zusätzlich auch eine mechanische Stoßbeziehungsweise Berührungsdämpfungslage des Formgestricks bilden kann.

In einer vorteilhaften Weiterbildung wird vorgeschlagen, dass das Formgestrick eine dritte Lage aufweist, in die mehrere linienartige oder flächenartige, insbesondere streifenförmige, elektrisch leitende Strukturen aus einem elektrisch leitfähigen Garn, vorzugsweise als Intarsiamuster oder eingeringelte Flächen, und linienartige oder flächenartige, insbesondere streifenförmige, elektrisch nichtleitende Strukturen aus einem elektrisch nicht leitfähigen Garn, vorzugsweise als Intarsiamuster oder eingeringelte Flächen, derart eingestrickt sind, dass die elektrisch leitenden Strukturen elektrisch voneinander isoliert sind, wobei jede der elektrisch leitenden Strukturen individuell elektrisch kontaktierbar und an eine Auswerteschaltung anschließbar ist. Dadurch ergeben sich im Vergleich zu einem einlagigen oder zweilagigen Formgestrick zusätzliche vorteilhafte Verwendungsmöglichkeiten für das Formgestrick.

Die elektrische Verschaltung/Kontaktierung der elektrisch leitenden Strukturen der ersten Lage und der dritten Lage mit der Auswerteschaltung kann sowohl durch die ein- und auslaufenden elektrisch leitfähigen Garne als auch durch das mustergesteuerte Einarbeiten von isolierten Mikrokabeln als Stehfäden oder eingelegte Flottfäden ermöglicht beziehungsweise erleichtert werden. Die punktuelle Kontaktierung der elektrisch leitfähigen Strickgarne mit isolierten Mikrokabeln oder isolierten leitfähigen Garnen kann nach dem definierten Abisolieren der Mikrokabel (beispielsweise durch Beaufschlagung mit Laserlicht) mittels Leitkleber, Löten oder durch Übernähen mit leitfähigem Nähgarn erfolgen.

Wenn die elektrisch leitenden Strukturen der ersten Lage und der dritten Lage linienartig oder flächenartig, insbesondere streifenförmig, ausgebildet sind, besteht die Möglichkeit, dass sich die linienartigen oder streifenförmigen elektrisch leitenden Strukturen der ersten Lage parallel zueinander in einer ersten Richtung erstrecken und dass sich die linienartigen oder streifenförmigen elektrisch leitenden Strukturen der dritten Lage parallel zueinander in einer zweiten Richtung erstrecken, die von der ersten Richtung verschieden ist. Die erste Richtung und die zweite Richtung können insbesondere zwei zueinander orthogonale Raumrichtungen sein und eine waagerechte und eine senkrechte Richtung des (flächigen) Formgestricks bilden. Dadurch wird in vorteilhafter Weise ein Formgestrick mit einer sich kreuzenden, matrixartigen Struktur der elektrisch leitenden Strukturen der ersten Lage und der elektrisch leitenden Strukturen der dritten Lage gebildet, so dass durch eine entsprechende individuelle elektrische Kontaktierung der Auswerteschaltung beispielsweise eine Sensoranordnung mit einer Ortsauflösung erhalten werden kann. Die matrixartige Struktur kann auch schiefwinklig oder freiformflächig gestaltet sein, wenn die erste Richtung und die zweite Richtung nicht orthogonal zueinander orientiert sind.

Das gemäß dieser Weiterbildung dreilagig ausgeführte Formgestrick kann somit zusammen mit der Auswerteschaltung eine Sensoranordnung bilden, die zumindest eine sich während einer äußeren Krafteinwirkung auf die Lagen ändernde elektrische Eigenschaft aufweist. Die Sensoranordnung kann - in Abhängigkeit von der Ausgestaltung der zweiten (mittleren) Lage - als kapazitive Sensoranordnung und/oder als piezoelektrische Sensoranordnung und/oder als resistive beziehungsweise piezoresistive Sensoranordnung ausgebildet sein. Vorzugsweise kann mittels der Sensoranordnung nicht nur das Vorhandensein einer von außen auf das Formgestrick einwirkenden Kraft, sondern auch die Größe der Kraft erfasst werden.

Als kapazitive Sensoranordnung funktioniert dieses dreilagige Formgestrick dann, wenn die beiden außen liegenden Elektroden, die durch die linienartigen oder flächenartigen, insbesondere streifenförmigen, elektrischen Strukturen der ersten und dritten Lage des Formgestricks gebildet sind, zusammen mit dem dazwischenliegenden Dielektrikum der zweiten Lage aus technisch-funktionaler Sicht einen Kondensator bilden, dessen Kapazität sich bei einer Krafteinwirkung durch eine Variation der räumlichen Form ändert. Diese Veränderung des elektrischen Feldes kann von der Auswerteeinheit der Auswerteschaltung, die an die durch die linienartigen oder flächenartigen, insbesondere streifenförmigen, elektrisch leitenden Strukturen gebildeten Elektroden der ersten und dritten Lage angeschlossen ist, mit Ortsauflösung erfasst und ausgewertet werden.

Resistiv beziehungsweise piezoresistiv funktioniert die Sensoranordnung mit den drei Lagen des Formgestricks dann, wenn die äußere Krafteinwirkung dazu führt, dass sich der innere elektrische Widerstand (Durchgangswiderstand) zwischen den beiden äußeren Elektroden bei einer von außen angelegten elektrischen Spannung in Abhängigkeit von der von außen einwirkenden Kraft verändert. Diese Änderung des Durchgangswiderstands kann mittels einer entsprechenden Auswerteeinheit der Auswerteschaltung bestimmt werden.

Umgekehrt kann eine derartige Sensoranordnung mit den drei Lagen des Formgestricks auch als piezoelektrischer Sensor verwendet werden, wenn sich bei einer äußeren Krafteinwirkung elektrische Spannungen zwischen den beiden Elektroden der äußeren Lagen ausbilden, die sich mit einer entsprechenden Auswerteeinheit der Auswerteschaltung messen lassen.

Je nach Ausbildung der Elektronik können diese unterschiedlichen, vorstehend erläuterten Sensorprinzipien auch kombiniert beziehungsweise sequentiell genutzt werden.

Die Verbindung der zweiten Lage mit der dritten Lage des Formgestricks kann vorzugsweise zum Beispiel durch ein textiles Verbindungsverfahren, insbesondere durch Stricken oder Nähen, oder auch durch ein stoffschlüssiges Verbindungsverfahren, insbesondere durch Verkleben, realisiert sein.

In einer besonders vorteilhaften Ausführungsform wird vorgeschlagen, dass alle drei Lagen des Formgestricks stricktechnisch hergestellt sind. Dadurch ergeben sich besondere Vorteile bei der Herstellung, da alle drei Lagen des Formgestricks in einem einzigen Strickprozess, insbesondere in einer Flachstrickvorrichtung, hergestellt werden können.

Die zweite (mittlere) Lage kann insbesondere aus einem Garn mit zwar vorhandener, jedoch lediglich geringer elektrischer Leitfähigkeit gestrickt sein. Beispielsweise kann die zweite Lage aus einem kohlenstoffgefüllten Garn hergestellt sein, welches seine elektrischen Eigenschaften, wie zum Beispiel seinen elektrischen Durchgangswiderstand, druckabhängig verändert. Das Garn, aus dem die zweite Lage gestrickt ist, kann alternativ zum Beispiel auch aus einem Polymer hergestellt sein, das mit einem elektrisch leitfähigen Material (insbesondere Ruß oder Metall) gefüllt ist oder aus einem intrinsisch leitfähigen Polymer besteht. Dieses ändert ebenfalls druckabhängig seinen Durchgangswiderstand.

In einer weiteren alternativen Ausführungsform besteht die Möglichkeit, dass das Garn, aus dem die zweite Lage gestrickt ist, eine drucksensitive, elektrisch leitfähige Beschichtung aufweist oder aus einem drucksensitiven Material hergestellt ist.

Wenn das dreilagige Formgestrick - wie oben erläutert - als resistive Sensoranordnung ausgebildet ist, können die elektrisch leitenden Strukturen der ersten und der dritten Lage vorzugsweise als flächenartige, insbesondere streifenförmige, Strukturen gestrickt werden, die durch schmale elektrisch nichtleitende Bereiche beziehungsweise Strukturen, die vorzugsweise ebenfalls linien- oder streifenförmig gestrickt sein können, elektrisch voneinander isoliert sind. Die zweite (mittlere) Lage wird vorzugsweise aus einem druckabhängig elektrisch leitfähigen Material gestrickt. Beim Aufeinanderstricken der drei Lagen wird damit eine Matrixstruktur erzeugt, die druckabhängige Signale ortsaufgelöst liefern kann.

Eine andere sensorische Variante besteht darin, dass die mit der ersten und mit der dritten Lage des Formgestricks verbundene zweite Lage nicht elektrisch leitend ist. Durch eine Druckbelastung beziehungsweise Annäherung ändert sich das elektrische Feld zwischen den elektrisch leitenden Strukturen der beiden äußeren Lagen. Wenn die zweite Lage somit aus einem elektrisch nicht leitfähigen Garn hergestellt ist, ergibt sich eine kapazitive Sensoranordnung. Die zweite, nicht elektrisch leitende Lage bildet dabei ein Dielektrikum, so dass das dreilagige Formgestrick eine kapazitive Sensoranordnung nach Art eines Plattenkondensators bildet.

Weiterhin besteht in einer vorteilhaften Ausführungsform die Möglichkeit, dass Aussparungen innerhalb des ein- oder mehrlagigen Formgestricks, die in technischen Geweben nicht oder nur mit einem großen Aufwand erzeugbar sind, geschnitten werden oder alternativ auch stricktechnisch hergestellt werden können.

In einer besonders vorteilhaften Ausführungsform kann vorgesehen sein, dass die Breite der streifenförmigen elektrisch leitenden Strukturen der ersten Lage und/oder die Breite der streifenförmigen elektrisch leitenden Strukturen der dritten Lage größer als die Breite der benachbarten, nichtleitenden Strukturen der betreffenden Lage ist. Durch diese Maßnahme wird in vorteilhafter Weise die Breite der elektrisch nichtleitenden Strukturen der ersten Lage und/oder der dritten Lage minimiert, so dass der sensorisch aktive Flächenanteil des Formgestricks entsprechend maximiert werden kann.

Mittels des hier vorgestellten Formgestricks wird somit eine annäherungssensitive und/oder drucksensitive, ortsauflösende sensorische (Ober-)Fläche geschaffen, die insbesondere im Flachstrickverfahren ohne großen Konfektionsaufwand für unterschiedliche, auch unregelmäßig geformte Körper hergestellt werden kann und die zum Beispiel dafür eingesetzt werden kann, um bei einer Mensch-Roboter-Kollaboration/Interaktion Berührungen zwischen Mensch und Roboter zu erkennen.

Eine weitere Art der berührungssensitiven Sensorik wird möglich, wenn die zweite Lage nicht als flächenartige Zwischenschicht ausgebildet ist, sondern eine Mehrzahl punktueller Abstandshalter zu der ersten Lage und/oder zu der dritten Lage umfasst. Die elektrisch leitenden Strukturen der ersten Lage und die elektrisch nichtleitenden Strukturen der dritten Lage weisen nach dem Anschluss an eine Spannungsversorgungseinrichtung der Auswerteschaltung ein unterschiedliches elektrisches Potential auf. Bei einer Druckeinwirkung wird der mechanische Widerstand der (insbesondere gestrickten) Fäden, welche die Abstandshalter bilden, überwunden, so dass die erste Lage und die dritte Lage miteinander in Kontakt kommen. Dadurch entsteht ein elektrisches Signal, das von der Auswerteeinheit der Auswerteschaltung wiederum mit Ortsauflösung erfasst werden kann.

Gemäß Anspruch 12 schlägt die Erfindung eine Verwendung eines Formgestricks nach einem der Ansprüche 1 bis 11 und einer daran angeschlossenen Auswerteschaltung als Sensoranordnung vor. Das erfindungsgemäße Formgestrick kann - wie oben bereits erwähnt - insbesondere als Sensoranordnung einer druckempfindlichen Schutzeinrichtung zur Überwachung einer technischen Anlage eingesetzt werden.

Darüber hinaus schlägt die Erfindung eine Verwendung eines Formgestricks nach einem der Ansprüche 1 bis 11 als Schaltvorrichtung und/oder als Eingabevorrichtung vor. Eine mögliche Applikation ist zum Beispiel eine druckempfindliche Eingabevorrichtung, mittels derer von einem Benutzer durch Drücken auf entsprechende Felder des mehrlagigen matrixartigen Formgestricks entsprechende Bedieneingaben vorgenommen werden können. Eine druckempfindliche Eingabetastatur lässt sich mittels des mehrlagigen matrixartigen Formgestricks ebenfalls sehr einfach realisieren.

Nachfolgend sollen einige Vorteile der hier vorgestellten Ausführungsformen des Formgestricks zusammengefasst werden:
- Die Drapierbarkeit ist erheblich höher als bei Geweben.
- Die Maschenstruktur ermöglicht eine Strukturverformung nicht nur in der Fläche, sondern auch bei räumlicher Umschließung eines mit dem Formgestrick versehenen Objekts.
- In der Herstellung können durch Weglassen von Maschen oder durch unterschiedliche Maschengrößen 2D- und 3D-Formgebungen erzielt werden. Dieses ist bei der Webtechnik so nicht möglich.
- Durch die Intarsienflächen ist eine freie Flächenbelegungsgestaltung für Funktionsbereiche - unabhängig von oberer oder unterer Lage des Formgestricks - möglich.
- Sortenreine Intarsienflächen sind möglich, ohne dass ein anderes Garnmaterial mitgeführt werden muss. Dieses ist beim Weben nicht möglich.
- Eine Flächenkontaktierung von elektrisch leitfähigen Fäden oder den alternativ verwendbaren dünnen metallischen Drähten/Mikrokabeln ist beim Stricken mit erheblich weniger Aufwand als beim Weben möglich.
- Ein Formgestrick weist eine höhere Weichheit als ein Gewebe auf.
- Ein Formgestrick weist bessere Stoßdämpfungsbeziehungsweise Berührungsdämpfungseigenschaften als ein Gewebe auf.
- Bei einer Verwendung des Formgestricks als sensorische zweidimensionale Roboterhaut ergibt sich eine zusätzliche Reduzierung von Verletzungsgefahren (letzter Notrückhalt, Aufnahme des Nachlaufs beim Roboterstop).

Weitere Anwendungsgebiete der vorliegenden Erfindung sind zum Beispiel:
- textile Taster beziehungsweise Schalter,
- taktile Sensorapplikationen,
- Applikationen mit Gestensteuerung,
- Sensoren und Einrichtungen zur Roboter-/Maschine-Mensch-Absicherung,
- Sitzbelegungserkennungs- und Schaltfunktionen in Fahrzeugen (Landfahrzeugen, Schienenfahrzeugen, Luftfahrzeugen oder Wasserfahrzeugen) oder in Möbeln,
- Positionserkennung und Lageerkennung (zum Beispiel einer gestürzten Person), insbesondere auf einem Teppich oder auf einem Teppichboden,
- Verhindern von Einklemm- und/oder Stoßsituationen bei Maschinenkomponenten, Gebäuden, Möbeln, Türen, Öffnungen etc.

Weitere Merkmale und Vorteile der vorliegenden Erfindung werden deutlich anhand der nachfolgenden Beschreibung bevorzugter Ausführungsbeispiele unter Bezugnahme auf die beiliegenden Abbildungen. Darin zeigen:
- Fig. 1: ein einlagiges Intarsia-Gestrick gemäß dem Stand der Technik mit elektrisch leitenden und elektrisch nichtleitenden Strukturen,
- Fig. 2: ein weiteres einlagiges Intarsia-Gestrick mit elektrisch leitenden und elektrisch nichtleitenden Strukturen gemäß dem Stand der Technik,
- Fig. 3: eine schematische Darstellung eines einlagigen Formgestricks, das gemäß einem ersten Ausführungsbeispiel der vorliegenden Erfindung ausgeführt ist,
- Fig. 4: eine schematische Darstellung eines einlagigen Formgestricks, das gemäß einem zweiten Ausführungsbeispiel der vorliegenden Erfindung ausgeführt ist,
- Fig. 5: eine schematische Darstellung eines einlagigen Formgestricks, das gemäß einem dritten Ausführungsbeispiel der vorliegenden Erfindung ausgeführt ist,
- Fig. 6: eine schematische Darstellung eines einlagigen Formgestricks, das gemäß einem vierten Ausführungsbeispiel der vorliegenden Erfindung ausgeführt ist,
- Fig. 7: eine perspektivische, auseinandergezogene Darstellung eines Formgestricks, das gemäß einem fünften Ausführungsbeispiel der vorliegenden Erfindung ausgeführt ist,
- Fig. 8: eine schematische Darstellung eines möglichen Aufbaus einer ersten Lage des Formgestricks gemäß Fig. 7,
- Fig. 9: eine schematische Darstellung eines möglichen Aufbaus einer dritten Lage des Formgestricks gemäß Fig. 7,
- Fig. 10: eine perspektivische, auseinandergezogene Darstellung eines Formgestricks, das gemäß einem sechsten Ausführungsbeispiel der vorliegenden Erfindung ausgeführt ist,
- Fig. 11: eine schematische Darstellung, die einen elektrischen Anschluss eines der Formgestricke gemäß Fig. 3 bis 6 an eine Auswerteschaltung veranschaulicht,
- Fig. 12: eine schematische Darstellung, die einen elektrischen Anschluss des Formgestricks gemäß Fig. 7 an eine Auswerteschaltung veranschaulicht.

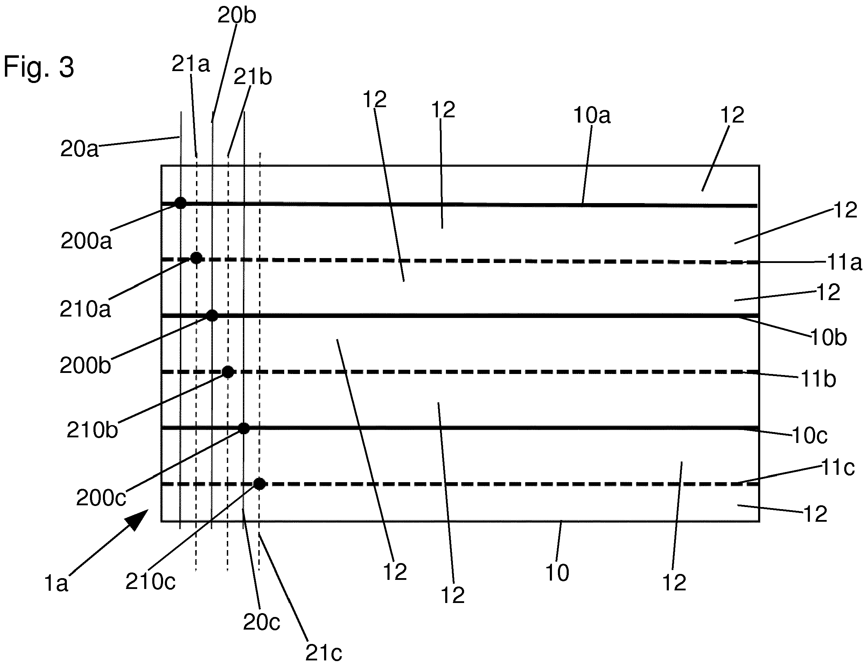

Unter Bezugnahme auf Fig. 3 soll nachfolgend ein Formgestrick 1a näher erläutert werden, das gemäß einem ersten Ausführungsbeispiel der vorliegenden Erfindung ausgeführt ist. Das Formgestrick 1a ist vorliegend als Ringelgestrick ausgebildet, welches eine erste Lage 10 mit elektrisch leitenden Bereichen und elektrisch nichtleitenden Bereichen beziehungsweise Strukturen 12 aufweist. Die elektrisch leitenden Bereiche werden in diesem und in den übrigen Ausführungsbeispielen aus einem elektrisch leitfähigen Garn hergestellt. Elektrisch leitfähige Garne können zum Beispiel aus einem Polymer, das mit einem elektrisch leitfähigen Material - insbesondere Ruß oder Metall - gefüllt ist, oder aus einem intrinsisch leitfähigen Polymer bestehen oder metallisch leitfähig sein oder mit leitfähigem Polymer beschichtet sein oder aus feinen Metalldrähten beziehungsweise Metalllitzen bestehen oder einen Kernfaden umfassen, der mit feinen Metalldrähten oder Metalllitzen umwunden ist.

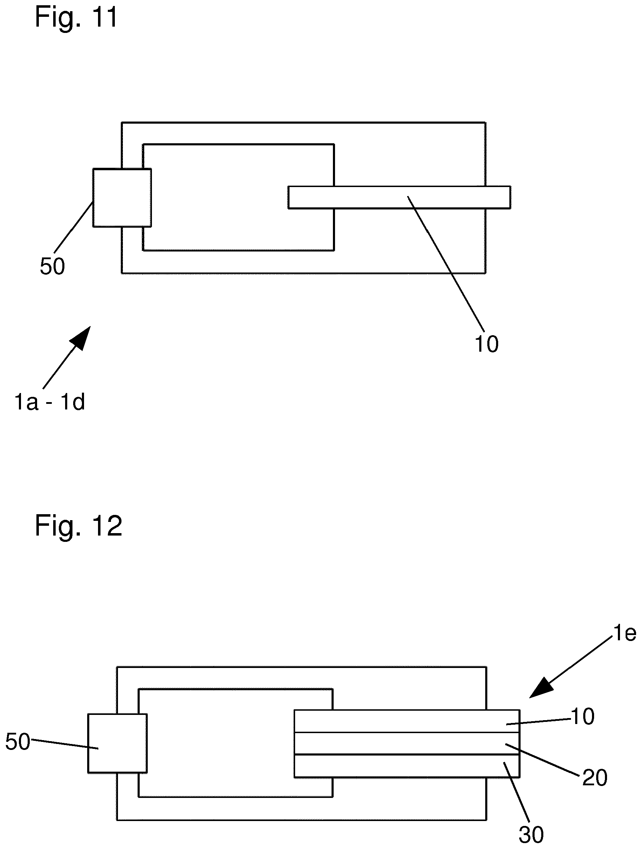

Das Formgestrick 1a weist mehrere, sich vorliegend in einer ersten Richtung (insbesondere einer waagerechten Richtung) erstreckende elektrisch leitende Strukturen 10a, 10b, 10c, 11a, 11b, 11c auf, die linienartig oder alternativ flächenartig, insbesondere streifenförmig, ausgebildet sind und aus elektrisch leitfähigen Garnen gestrickt sind. Die in Fig. 3 zur besseren Unterscheidung durchgehend beziehungsweise unterbrochen dargestellten (tatsächlich natürlich nicht unterbrochenen), in der ersten Richtung orientierten elektrisch leitenden Strukturen 10a, 10b, 10c, 11a, 11b, 11c bilden eine durchgängige elektrisch leitende Linien- beziehungsweise Streifenanordnung in der ersten Richtung, wobei benachbarte elektrisch leitende Strukturen 10a, 10b, 10c, 11a, 11b, 11c durch die dazwischenliegenden elektrisch nichtleitenden Bereiche beziehungsweise Strukturen 12 elektrisch voneinander isoliert sind. Die linienartig beziehungsweise flächenartig ausgebildeten elektrisch leitenden Strukturen 10a, 10b, 10c, 11a, 11b, 11c ermöglichen zum Beispiel eine Verwendung des Formgestricks 1a in einer Sensorapplikation zur Überwachung linienförmiger beziehungsweise streifenförmiger Bereiche in der ersten Richtung, in der sich die elektrisch leitenden Strukturen 10a, 10b, 10c, 11a, 11b, 11c erstrecken. Die elektrisch leitenden Strukturen 10a, 10b, 10c, 11a, 11b, 11c werden abwechselnd mit einem Pluspol und mit einem Minuspol einer hier nicht explizit dargestellten elektrischen Spannungsversorgungseinrichtung einer Auswerteschaltung 50 kontaktiert. Die an das Formgestrick 1a angeschlossene Auswerteschaltung 50 ist in Fig. 11 dargestellt. Dabei handelt es sich um eine Möglichkeit, aber nicht um die einzige Möglichkeit, das Formgestrick 1a an die Auswerteschaltung 50 anzuschließen. Die elektrische Verschaltung hängt insbesondere von der Art der Verwendung des Formgestricks 1a ab.

Somit bilden die elektrisch leitenden Strukturen 10a, 10b, 10c des Formgestricks 1a eine erste Gruppe, die mit dem Pluspol der Spannungsversorgungeinrichtung der Auswerteschaltung 50 verbunden ist. Demgegenüber bilden die elektrisch leitenden Strukturen 11a, 11b, 11c eine zweite Gruppe, die mit dem Minuspol der Spannungsversorgungeinrichtung verbunden ist. Die individuelle elektrische Kontaktierung der elektrisch leitenden Strukturen 10a, 10b, 10c der ersten Gruppe mit dem Pluspol der Spannungsversorgungseinrichtung der Auswerteschaltung 50 und der elektrisch leitenden Strukturen 11a, 11b, 11c der zweiten Gruppe mit dem Minuspol der Spannungsversorgungseinrichtung der Auswerteschaltung 50 kann zum Beispiel mittels eines mehradrigen elektrischen Anschlusskabels erfolgen.

Die individuelle elektrische Kontaktierung - abwechselnd Pluspol und Minuspol - der elektrisch leitenden Strukturen 10a, 10b, 10c, 11a, 11b, 11c erfolgt vorliegend insbesondere über isolierte Mikrokabel oder isolierte leitfähige Garne oder Umwindegarne, die als Stehfäden 20a, 20b, 20c, 21a, 21b, 21c ausgebildet sind, welche sich in einer zweiten Richtung (parallel zu den Maschenstäbchen) und somit orthogonal zur ersten Richtung erstrecken. Die Stehfäden 20a, 20b, 20c, 21a, 21b, 21c sind dabei in die erste Lage 10 des Formgestricks 1a eingelegt, ohne dass sie zu Maschen geformt sind. Diese Stehfäden 20a, 20b, 20c, 21a, 21b, 21c werden partiell, zum Beispiel mittels Laserlicht, abisoliert und jeweils über eine elektrisch leitfähige Verbindung 200a, 200b, 200c, 210a, 210b, 210c mit einer der elektrisch leitenden Strukturen 10a, 10b, 10c, 11a, 11b, 11c der ersten Lage 10 des Formgestricks 1a verbunden und ermöglichen dadurch eine individuelle elektrische Kontaktierung der elektrisch leitenden Strukturen 10a, 10b, 10c, 11a, 11b, 11c. Die elektrisch leitfähigen Verbindungen 200a, 200b, 200c, 210a, 210b, 210c können zum Beispiel durch Vernähen mit einem elektrisch leitfähigen Nähgarn, durch Verkleben mit einem elektrisch leitfähigen Kleber oder durch Vercrimpen erhalten werden.

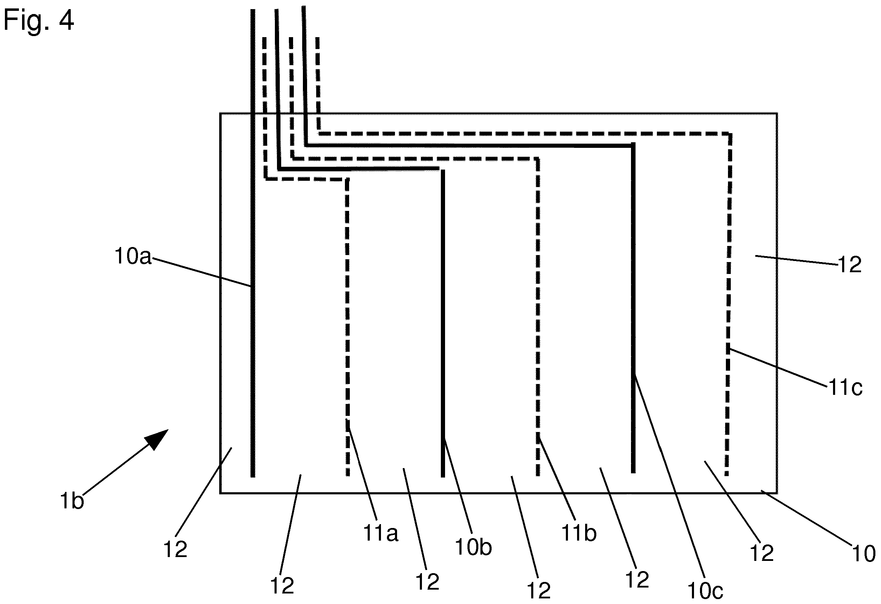

Fig. 4 zeigt ein Formgestrick 1b, das gemäß einem zweiten Ausführungsbeispiel der vorliegenden Erfindung ausgeführt ist. Das Formgestrick 1b ist hierbei als Intarsia-Gestrick ausgebildet, welches eine erste Lage 10 mit elektrisch leitenden Bereichen und elektrisch nichtleitenden Bereichen beziehungsweise Strukturen 12 aufweist. Das Formgestrick 1b weist mehrere, sich vorliegend zumindest abschnittsweise in einer zweiten Richtung (senkrechten Richtung) erstreckende elektrisch leitende Strukturen 10a, 10b, 10c, 11a, 11b, 11c auf, die linienartig oder alternativ flächenartig, insbesondere streifenförmig, ausgebildet sind und aus elektrisch leitfähigen Garnen gestrickt sind. Die in Fig. 4 wiederum durchgehend beziehungsweise unterbrochen dargestellten, zumindest abschnittsweise in der zweiten (senkrechten) Richtung, die sich orthogonal zu der ersten Richtung gemäß Fig. 3 erstreckt, orientierten elektrisch leitenden Strukturen 10a, 10b, 10c, 11a, 11b, 11c bilden jeweils eine durchgängige Linienbeziehungsweise Streifenanordnung, wobei benachbarte elektrisch leitende Strukturen 10a, 10b, 10c, 11a, 11b, 11c elektrisch voneinander isoliert sind. Die linienartig beziehungsweise flächenartig ausgebildeten elektrisch leitenden Strukturen 10a, 10b, 10c, 11a, 11b, 11c ermöglichen zum Beispiel eine Verwendung des Formgestricks 1a in einer Sensorapplikation zur Überwachung linienförmiger bzw. streifenförmiger Bereiche in der zweiten (senkrechten) Richtung, in der sich die elektrisch leitenden Strukturen 10a, 10b, 10c, 11a, 11b, 11c in diesem Ausführungsbeispiel abschnittsweise erstrecken. Die elektrisch leitenden Strukturen 10a, 10b, 10c, 11a, 11b, 11c werden abwechselnd mit einem Pluspol und mit einem Minuspol einer elektrischen Spannungsversorgungseinrichtung der Auswerteschaltung 50 kontaktiert. Somit bilden die elektrisch leitenden Strukturen 10a, 10b, 10c eine erste Gruppe, die mit dem Pluspol der elektrischen Spannungsversorgungseinrichtung der Auswerteschaltung 50 verbunden ist. Demgegenüber bilden die elektrisch leitenden Strukturen 11a, 11b, 11c eine zweite Gruppe, die mit dem Minuspol der elektrischen Spannungsversorgungseinrichtung der Auswerteschaltung 50 verbunden ist. Die individuelle elektrische Kontaktierung - abwechselnd Pluspol und Minuspol - erfolgt hierbei direkt über die in das Formgestrick 1b eingestrickten elektrisch leitfähigen Garne, aus denen die elektrisch leitenden Strukturen 10a, 10b, 10c, 11a, 11b, 11c gebildet sind.

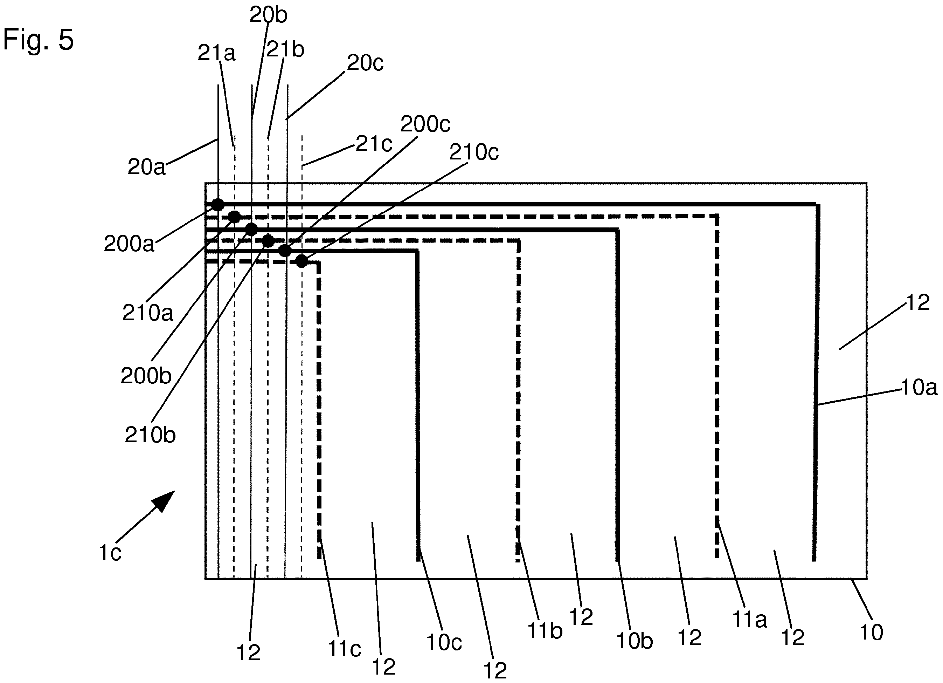

Unter Bezugnahme auf Fig. 5 soll nachfolgend ein einlagiges Formgestrick 1c näher erläutert werden, das gemäß einem dritten Ausführungsbeispiel der vorliegenden Erfindung ausgeführt ist. Das Formgestrick 1c ist als Gestrick mit senkrecht eingelegten Stehfäden ausgebildet, welches eine erste Lage 10 mit elektrisch leitenden Bereichen und elektrisch nichtleitenden Bereichen beziehungsweise Strukturen 12 aufweist. Das Formgestrick 1c weist mehrere, sich vorliegend zumindest abschnittsweise in der zweiten (senkrechten) Richtung erstreckende elektrisch leitende Strukturen 10a, 10b, 10c, 11a, 11b, 11c auf, die linienartig oder alternativ flächenartig, insbesondere streifenförmig, ausgebildet sind und aus elektrisch leitfähigen Garnen bestehen. Die in Fig. 5 durchgehend beziehungsweise unterbrochen dargestellten, zumindest abschnittsweise in der zweiten (senkrechten) Richtung orientierten elektrisch leitenden Strukturen 10a, 10b, 10c, 11a, 11b, 11c bilden jeweils eine durchgängige Linien- beziehungsweise Streifenanordnung, wobei benachbarte elektrisch leitende Strukturen 10a, 10b, 10c, 11a, 11b, 11c durch die gestrickten nicht elektrisch leitenden Strukturen 12 elektrisch voneinander isoliert sind. Die linienartig beziehungsweise flächenartig ausgebildeten elektrisch leitenden Strukturen 10a, 10b, 10c, 11a, 11b, 11c ermöglichen zum Beispiel eine Verwendung des Formgestricks 1a in einer Sensorapplikation zur Überwachung linienförmiger bzw. streifenförmiger Bereiche in der zweiten (senkrechten) Richtung, in der sich die elektrisch leitenden Strukturen 10a, 10b, 10c, 11a, 11b, 11c erstrecken. Die elektrisch leitenden Strukturen 10a, 10b, 10c, 11a, 11b, 11c werden individuell abwechselnd mit einem Pluspol und mit einem Minuspol einer elektrischen Spannungsversorgungseinrichtung der Auswerteschaltung 50 kontaktiert. Somit bilden die elektrisch leitenden Strukturen 10a, 10b, 10c wiederum eine erste Gruppe, die mit dem Pluspol verbunden ist. Demgegenüber bilden die elektrisch leitenden Strukturen 11a, 11b, 11c eine zweite Gruppe, die mit dem Minuspol verbunden ist.

Die individuelle elektrische Kontaktierung - abwechselnd Pluspol und Minuspol - der elektrisch leitenden Strukturen 10a, 10b, 10c, 11a, 11b, 11c erfolgt hierbei wie im ersten Ausführungsbeispiel durch isolierte Mikrokabel oder isolierte elektrisch leitfähige Garne oder Umwindegarne, die als Stehfäden 20a, 20b, 20c, 21a, 21b, 21c ausgebildet sind, welche sich in der zweiten, vorliegend senkrechten Richtung (parallel zu den Maschenstäbchen) erstrecken. Die Stehfäden 20a, 20b, 20c, 21a, 21b, 21c sind dabei wiederum in die erste Lage 10 des Formgestricks 1a eingelegt, ohne dass sie zu Maschen geformt sind. Diese Stehfäden 20a, 20b, 20c, 21a, 21b, 21c werden partiell, zum Beispiel mittels Laserlicht, abisoliert und jeweils mit einer elektrisch leitfähigen Verbindung 200a, 200b, 200c, 210a, 210b, 210c mit einer der elektrisch leitenden Strukturen 10a, 10b, 10c, 11a, 11b, 11c verbunden und dadurch elektrisch kontaktiert. Die elektrisch leitfähigen Verbindungen 200a, 200b, 200c, 210a, 210b, 210c können zum Beispiel durch Vernähen mit einem leitfähigen Nähgarn, durch Verkleben mit einem elektrisch leitfähigen Kleber oder durch Vercrimpen erhalten werden.

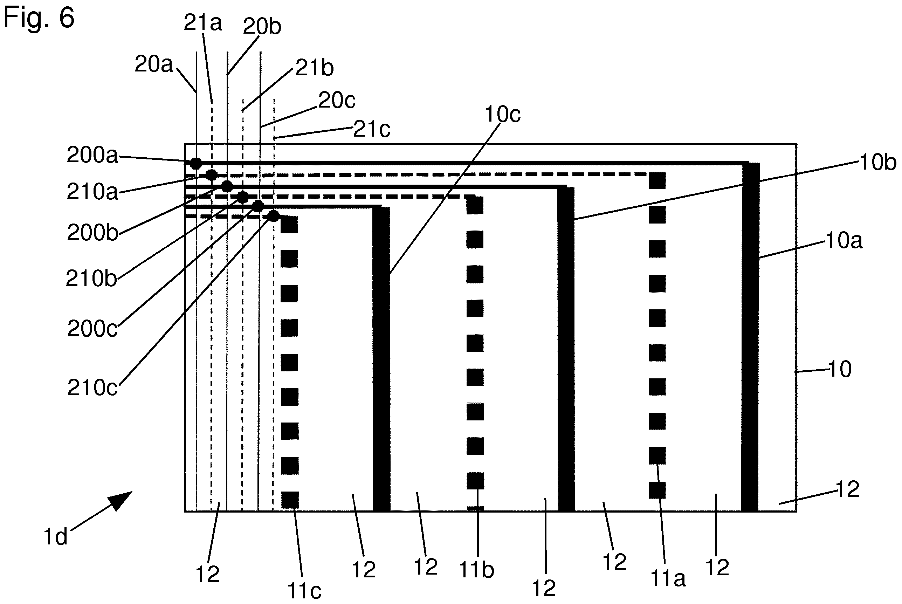

Unter Bezugnahme auf Fig. 6 soll nachfolgend ein einlagiges Formgestrick 1d näher erläutert werden, welches gemäß einem vierten Ausführungsbeispiel der vorliegenden Erfindung ausgeführt ist. Dieses stellt eine Weiterbildung des in Fig. 5 dargestellten Ausführungsbeispiels dar. Der grundlegende Aufbau des Formgestricks 1d im Hinblick auf die Anordnung der elektrisch leitenden Strukturen 10a, 10b, 10c, 11a, 11b, 11c in der ersten Lage 10 sowie die elektrische Kontaktierung entspricht demjenigen des in Fig. 5 dargestellten Ausführungsbeispiels. Die sich in der zweiten Richtung (senkrechten Richtung) erstreckenden Abschnitte der elektrisch leitenden Strukturen 10a, 10b, 10c, 11a, 11b, 11c sind in diesem Ausführungsbeispiel als Intarsiaflächen mit einer Breite von einer oder mehreren Maschen ausgebildet.

Jedes der vorstehend beschriebenen einlagigen Formgestricke 1a, 1b, 1c, 1d kann insbesondere in Sensorapplikationen eingesetzt werden. Zu diesem Zweck kann das einlagige Formgestrick 1a, 1b, 1c, 1d zur Bildung einer Sensoranordnung in geeigneter Weise an eine elektrische Spannungsversorgungseinrichtung der Auswerteschaltung 50 angeschlossen werden, so dass die einzelnen elektrisch leitenden Strukturen 10a, 10b, 10c, 11a, 11b, 11c des Formgestricks 1a, 1b, 1c, 1d individuell elektrisch kontaktiert werden können. Mit anderen Worten weist das einlagige Formgestrick 1a, 1b, 1c, 1d somit partielle Elektrodenflächen auf, die individuell elektrisch kontaktiert werden können und aufgrund der an diesen anliegenden elektrischen Spannungen elektrische Felder zueinander ausbilden und somit eine Annäherung von Gegenständen und Personen beziehungsweise Körperteilen erfassen können.

Mittels einer entsprechenden Auswerteeinheit der Auswerteschaltung 50, die an die elektrisch leitenden Strukturen 10a, 10b, 10c, 11a, 11b, 11c angeschlossen ist, können bei einer Annäherung zum Beispiel die Änderungen der elektrischen Felder erfasst werden. Jedes der vorstehend beschriebenen einlagigen Formgestricke 1a, 1b, 1c, 1d kann somit insbesondere als kapazitiver Annäherungssensor einer Schutzeinrichtung zur Überwachung einer technischen Anlage verwendet werden.

In weiteren vorteilhaften, hier nicht explizit dargestellten Ausführungsformen, welche die Funktionalitäten der Formgestricke 1a, 1b, 1c, 1d erweitern können, besteht die Möglichkeit, dass die vorstehend beschriebenen Formgestricke 1a, 1b, 1c, 1d zumindest eine zweite Lage aufweisen, die mit der ersten Lage 10 verbunden ist. Die zweite Lage kann zum Beispiel ebenfalls eine gestrickte Lage sein, die stricktechnisch mit der ersten Lage 10 verbunden ist. Dadurch ergeben sich erhebliche Herstellungsvorteile, da die beiden Lagen des Formgestricks 1a, 1b, 1c, 1d in einem einzigen Strickprozess, insbesondere in einer Flachstrickvorrichtung, hergestellt werden können. Alternativ kann die zweite Lage auch mit der ersten Lage 10 vernäht sein. Die zweite Lage kann vollständig oder alternativ auch nur abschnittsweise aus einem Gestrick bestehen.

In alternativen Ausführungsformen kann die zweite Lage auch ein Gewebe und/oder ein Gewirk und/oder ein Gelege und/oder ein Vliesmaterial und/oder ein Schaummaterial und/oder eine Folie umfassen oder vollständig daraus bestehen. Die in dieser Weise ausgeführte zweite Lage kann zum Beispiel durch ein textiles Verbindungsverfahren, insbesondere durch Vernähen, oder auch stoffschlüssig, insbesondere durch Verkleben, mit der ersten Lage verbunden werden.

Die zweite Lage kann zum Beispiel elektrisch nichtleitend ausgebildet sein und aus technisch-funktionaler Sicht einen Berührungsschutz für die erste Lage 10 und/oder eine Isolation für die elektrisch leitenden Strukturen 10a, 10b, 10c, 11a, 11b, 11c der ersten Lage 10 bilden. Wenn die zweite Lage aus einem elastisch verformbaren Werkstoff hergestellt ist, kann diese zusätzlich auch eine mechanische Stoßbeziehungsweise Berührungsdämpfungslage des Formgestricks 1a, 1b, 1c, 1d bilden.

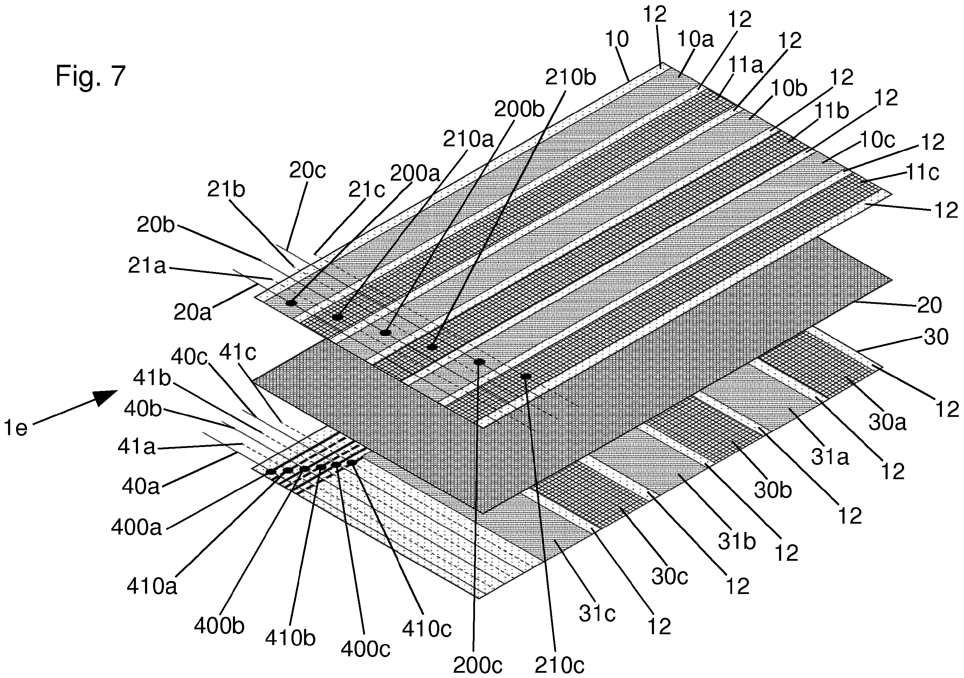

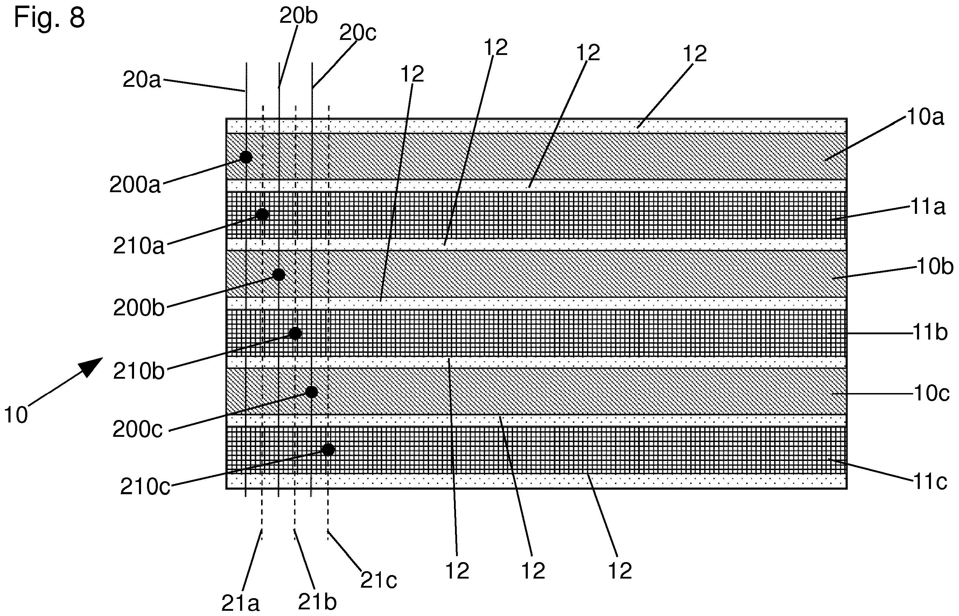

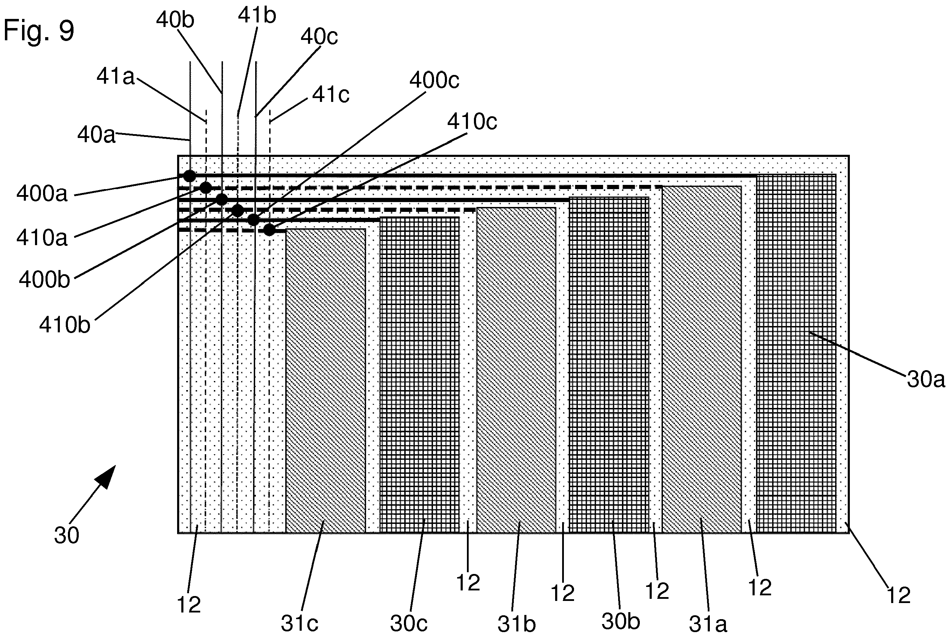

Unter Bezugnahme auf Fig. 7 soll nachfolgend ein fünftes Ausführungsbeispiel eines Formgestricks 1e näher erläutert werden. Dieses Formgestrick 1e ist dreilagig ausgeführt und weist eine erste Lage 10, eine zweite Lage 20 und eine dritte Lage 30 auf, die in Fig. 7 schematisch dargestellt sind. Fig. 8 zeigt einen möglichen Aufbau der ersten Lage 10. Ein möglicher Aufbau der dritten Lage 30 ist in Fig. 9 gezeigt. Fig. 12 zeigt das Formgestrick 1e nach dem Anschluss an eine Auswerteschaltung 50. Dabei handelt es sich um eine Möglichkeit, aber nicht um die einzige Möglichkeit, das Formgestrick 1e an die Auswerteschaltung 50 anzuschließen. Die elektrische Verschaltung hängt insbesondere von der Art der Verwendung des Formgestricks 1e ab.

Die erste Lage 10 des Formgestricks 1e ist vorliegend als Ringelgestrick ausgebildet und weist elektrisch leitende Bereiche und elektrisch nichtleitende Bereiche beziehungsweise Strukturen 12 auf. Die elektrisch leitenden Bereiche werden wiederum aus einem elektrisch leitfähigen Garn der oben bereits erläuterten Art hergestellt. Die erste Lage 10 des Formgestricks 1e weist mehrere, sich vorliegend in einer ersten Richtung (waagerechten Richtung) erstreckende elektrisch leitende Strukturen 10a, 10b, 10c, 11a, 11b, 11c auf, die flächenartig, insbesondere streifenförmig, ausgebildet sind und aus elektrisch leitfähigen Garnen gestrickt sind. Vorliegend sind die elektrisch leitenden Strukturen 10a, 10b, 10c, 11a, 11b, 11c als eingeringelte Flächen ausgebildet. Die in Fig. 8 durchgehend beziehungsweise unterbrochen dargestellten, in der ersten (waagerechten) Richtung orientierten elektrisch leitenden Strukturen 10a, 10b, 10c, 11a, 11b, 11c bilden eine durchgängige Streifenanordnung in der ersten Richtung, wobei benachbarte elektrisch leitende Strukturen 10a, 10b, 10c, 11a, 11b, 11c durch die gestrickten, elektrisch nichtleitenden Bereiche beziehungsweise Strukturen 12 elektrisch voneinander isoliert sind. Die elektrisch leitenden Strukturen 10a, 10b, 10c, 11a, 11b, 11c, die individuell elektrisch kontaktierbar sind, können zum Beispiel abwechselnd mit einem Pluspol und mit einem Minuspol einer elektrischen Spannungsversorgungseinrichtung der Auswerteschaltung 50 kontaktiert sein. Somit bilden die elektrisch leitenden Strukturen 10a, 10b, 10c eine erste Gruppe, die mit dem Pluspol der elektrischen Spannungsversorgungseinrichtung verbunden ist. Demgegenüber bilden die elektrisch leitenden Strukturen 11a, 11b, 11c eine zweite Gruppe, die mit dem Minuspol der elektrischen Spannungsversorgungseinrichtung verbunden ist.

Die individuelle elektrische Kontaktierung - abwechselnd Pluspol und Minuspol - der elektrisch leitenden Strukturen 10a, 10b, 10c, 11a, 11b, 11c erfolgt hierbei wiederum über isolierte Mikrokabel oder isolierte leitfähige Garne oder Umwindegarne, die als Stehfäden 20a, 20b, 20c, 21a, 21b, 21c ausgebildet sind, welche sich in einer zweiten, vorliegend senkrechten Richtung (parallel zu den Maschenstäbchen) erstrecken. Die Stehfäden 20a, 20b, 20c, 21a, 21b, 21c sind dabei in die erste Lage 10 des Formgestricks 1e eingelegt, ohne dass sie zu Maschen geformt sind. Diese Stehfäden 20a, 20b, 20c, 21a, 21b, 21c werden partiell, zum Beispiel mittels Laserlicht, abisoliert und jeweils mit einer elektrisch leitfähigen Verbindung 200a, 200b, 200c, 210a, 210b, 210c mit einer der elektrisch leitenden Strukturen 10a, 10b, 10c, 11a, 11b, 11c verbunden und dadurch elektrisch kontaktiert. Die elektrisch leitfähigen Verbindungen 200a, 200b, 200c, 210a, 210b, 210c können zum Beispiel durch Vernähen mit einem leitfähigen Nähgarn, durch Verkleben mit einem elektrisch leitfähigen Kleber oder durch Vercrimpen erhalten werden.

Unter Bezugnahme auf Fig. 9 soll nachfolgend ein möglicher Aufbau einer dritten Lage 30 des dreilagigen Formgestrick 1e näher erläutert werden. Die dritte Lage 30 ist wiederum als Intarsia-Gestrick ausgebildet, welches elektrisch leitende Bereiche und elektrisch nichtleitende Bereiche 12 aufweist. Die dritte Lage 30 weist mehrere, sich vorliegend zumindest abschnittsweise in einer zweiten Richtung (senkrechten Richtung) erstreckende elektrisch leitende Strukturen 30a, 30b, 30c, 31a, 31b, 31c auf, die in diesem Ausführungsbeispiel flächenartig, insbesondere streifenförmig, ausgebildet sind und aus elektrisch leitfähigen Garnen gestrickt sind. Vorzugsweise sind die elektrisch leitenden Strukturen 30a, 30b, 30c, 31a, 31b, 31c als Intarsiaflächen mit einer Breite von einer oder mehreren Maschen ausgebildet.

Die in Fig. 9 durchgehend beziehungsweise unterbrochen dargestellten, zumindest abschnittsweise in der senkrechten Richtung orientierten elektrisch leitenden Strukturen 30a, 30b, 30c, 31a, 31b, 31c der dritten Lage 30 bilden jeweils eine durchgängige Streifenanordnung, wobei benachbarte elektrisch leitende Strukturen 30a, 30b, 30c, 31a, 31b, 31c durch die gestrickten nichtleitenden Bereiche 12 elektrisch voneinander isoliert sind. Die elektrisch leitenden Strukturen 30a, 30b, 30c, 31a, 31b, 31c, die individuell elektrisch kontaktierbar sind, werden abwechselnd mit einem Pluspol und mit einem Minuspol der elektrischen Spannungsversorgungseinrichtung der Auswerteschaltung 50 kontaktiert. Somit bilden die elektrisch leitenden Strukturen 30a, 30b, 30c eine erste Gruppe, die mit dem Pluspol verbunden ist. Demgegenüber bilden die elektrisch leitenden Strukturen 31a, 31b, 31c eine zweite Gruppe, die mit dem Minuspol verbunden ist.

Die elektrische Kontaktierung - abwechselnd Pluspol und Minuspol - der elektrisch leitenden Strukturen 30a, 30b, 30c, 31a, 31b, 31c der dritten Lage 30 erfolgt hierbei wie im ersten Ausführungsbeispiel durch isolierte Mikrokabel oder isolierte leitfähige Garne oder Umwindegarne, die als Stehfäden 40a, 40b, 40c, 41a, 41b, 41c ausgebildet sind, welche sich in einer zweiten, vorliegend senkrechten Richtung (parallel zu den Maschenstäbchen) erstrecken. Die Stehfäden 40a, 40b, 40c, 41a, 41b, 41c sind dabei in die dritte Lage 30 des Formgestricks 1e eingelegt, ohne dass sie zu Maschen geformt sind. Diese Stehfäden 40a, 40b, 40c, 41a, 41b, 41c werden partiell, zum Beispiel mittels Laserlicht, abisoliert und jeweils über eine elektrisch leitfähige Verbindung 400a, 400b, 400c, 410a, 410b, 410c mit einer der elektrisch leitenden Strukturen 30a, 30b, 30c, 31a, 31b, 31c verbunden und dadurch elektrisch kontaktiert. Die elektrisch leitfähigen Verbindungen 400a, 400b, 400c, 410a, 410b, 410c können zum Beispiel durch Vernähen mit einem leitfähigen Nähgarn, durch Verkleben mit einem elektrisch leitfähigen Kleber oder durch Vercrimpen erhalten werden.

Aus den vorhergehenden Erläuterungen wird deutlich, dass sich die streifenförmigen elektrisch leitenden Strukturen 10a, 10b, 10c, 11a, 11b, 11c der ersten Lage 10 des dreilagigen Formgestricks 1e parallel zueinander in einer ersten Richtung erstrecken, wohingegen sich die streifenförmigen elektrisch leitenden Strukturen 30a, 30b, 30c, 31a, 31b, 31c der dritten Lage 30 parallel zueinander in einer zweiten Richtung erstrecken, die von der ersten Richtung verschieden ist. Die erste Richtung und die zweite Richtung sind zwei zueinander orthogonale Raumrichtungen und bilden vorliegend eine senkrechte und eine waagerechte Richtung des Formgestricks 1e. Dadurch wird eine matrixartige Struktur der elektrisch leitenden, individuell kontaktierbaren Strukturen 10a, 10b, 10c, 11a, 11b, 11c der ersten Lage 10 und der elektrisch leitenden, individuell kontaktierbaren Strukturen 30a, 30b, 30c, 31a, 31b, 31c der dritten Lage 30 gebildet, so dass eine Sensoranordnung mit einer Ortsauflösung in der ersten Richtung und in der zweiten Richtung erhalten werden kann. Diese matrixartige Struktur kann alternativ auch schiefwinklig oder freiformflächig gestaltet sein.

Die Verbindung der zweiten Lage 20 mit der dritten Lage 30 des Formgestricks 1e kann zum Beispiel durch ein textiles Verbindungsverfahren, insbesondere durch Stricken oder Nähen, oder auch stoffschlüssig, insbesondere durch Verkleben, realisiert sein. Wenn alle drei Lagen 10, 20, 30 stricktechnisch hergestellt sind, ergeben sich besondere Vorteile bei der Herstellung, da alle drei Lagen 10, 20, 30 des Formgestricks 1e in einem einzigen Strickprozess, insbesondere in einer Flachstrickvorrichtung, hergestellt werden können.

Die zweite (mittlere) Lage 20 des Formgestricks 1e kann insbesondere aus einem Garn mit zwar vorhandener, jedoch lediglich geringer elektrischer Leitfähigkeit gestrickt sein. Beispielsweise kann die zweite Lage 20 aus einem kohlenstoffgefüllten Garn hergestellt sein, das seine elektrischen Eigenschaften, wie zum Beispiel seinen elektrischen Durchgangswiderstand, druckabhängig verändert. Das Garn, aus dem die zweite Lage 20 gestrickt ist, kann zum Beispiel auch aus einem Polymer hergestellt sein, das mit einem elektrisch leitfähigen Material (insbesondere Ruß oder Metall) gefüllt ist oder aus einem intrinsisch leitfähigen Polymer besteht. Dieses ändert ebenfalls druckabhängig seinen Durchgangswiderstand. In einer weiteren alternativen Ausführungsform besteht die Möglichkeit, dass das Garn, aus dem die zweite Lage 20 gestrickt ist, eine drucksensitive, elektrisch leitfähige Beschichtung aufweist oder aus einem drucksensitiven Material hergestellt ist.

Das dreilagige Formgestrick 1e gemäß dem dritten Ausführungsbeispiel kann somit in einer Sensoranordnung verwendet werden, die zumindest eine sich durch eine äußere Krafteinwirkung auf die Lagen 10, 20, 30 ändernde elektrische Eigenschaft aufweist. Die Sensoranordnung kann - abhängig von der Ausgestaltung der zweiten (mittleren) Lage 20 - insbesondere als kapazitive Sensoranordnung und/oder als piezoelektrische Sensoranordnung und/oder als resistive beziehungsweise piezoresistive Sensoranordnung ausgebildet sein. Vorzugsweise kann nicht nur das Vorhandensein einer von außen auf das Formgestrick 1e einwirkenden Kraft, sondern auch die Größe dieser Kraft (beziehungsweise des daraus resultierenden Drucks) erfasst werden.

Als kapazitive Sensoranordnung funktioniert dieses mehrlagige Formgestrick 1e dann, wenn die beiden außenliegenden Elektroden, die durch die elektrisch leitenden Strukturen 10a, 10b, 10c, 11a, 11b, 11c der ersten Lage 10 und durch die elektrisch leitenden Strukturen 30a, 30b, 30c, 31a, 31b, 31c der dritten Lage 30 gebildet sind, zusammen mit einem dazwischenliegenden Dielektrikum, welches durch die zweite Lage 20 gebildet ist, einen Kondensator bilden, dessen Kapazität sich bei einer äußeren Krafteinwirkung durch eine Variation der räumlichen Form ändert. Diese Veränderung des elektrischen Feldes kann von einer an das Formgestrick 1e angeschlossenen Auswerteeinheit der Auswerteschaltung 50 erfasst und mit Ortsauflösung ausgewertet werden.

Resistiv beziehungsweise piezoresistiv funktioniert die Sensoranordnung mit dem dreilagigen Formgestrick 1e dann, wenn sich der innere elektrische Widerstand des Formgestricks 1e zwischen den beiden äußeren Elektroden, die durch die elektrisch leitenden Strukturen 10a, 10b, 10c, 11a, 11b, 11c der ersten Lage 10 und durch die elektrisch leitenden Strukturen 30a, 30b, 30c, 31a, 31b, 31c der dritten Lage 30 gebildet sind, in Abhängigkeit von der von außen einwirkenden Kraft verändert.

Umgekehrt kann das Formgestrick 1e auch als piezoelektrische Sensoranordnung eingesetzt werden, wenn sich bei einer Krafteinwirkung elektrische Spannungen zwischen den beiden äußeren Elektroden, die durch die elektrisch leitenden Strukturen 10a, 10b, 10c, 11a, 11b, 11c der ersten Lage 10 und durch die elektrisch leitenden Strukturen 30a, 30b, 30c, 31a, 31b, 31c der dritten Lage 30 gebildet sind, ausbilden und mittels einer geeigneten Auswerteeinheit der Auswerteschaltung 50 messen lassen.

Diese unterschiedlichen Messverfahren können vorzugsweise auch kombiniert beziehungsweise sequentiell genutzt werden.

Wenn das dreilagige Formgestrick 1e - wie vorstehend erläutert - als resistive Sensoranordnung ausgebildet ist, können die elektrisch leitenden Strukturen 10a, 10b, 10c, 11a, 11b, 11c der ersten Lage 10 und die elektrisch leitenden Strukturen 30a, 30b, 30c, 31a, 31b, 31c der dritten Lage 30 vorzugsweise als flächenartige, insbesondere streifenförmige, Strukturen gestrickt werden, die durch schmale elektrisch nichtleitende Strukturen 12, die vorzugsweise ebenfalls linien- oder streifenförmig gestrickt sein können, elektrisch voneinander isoliert sind. Die zweite (mittlere) Lage 20 wird aus einem druckabhängig leitfähigen Material gestrickt. Beim Aufeinanderstricken der drei Lagen 10, 20, 30 wird damit eine Matrixstruktur erzeugt, die ortsaufgelöst druckabhängige Signale liefert.

Eine andere sensorische Variante besteht darin, dass die mit der ersten Lage 10 und der dritten Lage 30 verbundene zweite Lage 20 nicht elektrisch leitend ist. Durch eine Druckbelastung beziehungsweise Annäherung ändert sich das elektrische Feld zwischen den elektrischen Strukturen 10a, 10b, 10c, 11a, 11b, 11c der ersten Lage 10 und den elektrisch leitenden Strukturen 30a, 30b, 30c, 31a, 31b, 31c der dritten Lage 30, welche die beiden äußeren Lagen des Formgestricks 1e bilden. Wenn die zweite Lage 20 somit aus einem elektrisch nicht leitfähigen Garn hergestellt ist, ergibt sich eine kapazitive Sensoranordnung. Die zweite, nicht elektrisch leitende Lage 20 bildet dabei ein Dielektrikum, so dass das dreilagige Formgestrick 1e eine kapazitive Sensoranordnung nach Art eines Plattenkondensators bildet.

Vorzugsweise ist die Breite der elektrisch leitenden, streifenförmigen Strukturen 10a, 10b, 10c, 11a, 11b, 11c der ersten Lage 10 und/oder die Breite der elektrisch leitenden, streifenförmigen Strukturen 30a, 30b, 30c, 31a, 31b, 31c der dritten Lage 30 größer als die Breite der benachbarten, nichtleitenden streifenförmigen Strukturen 12 der betreffenden Lagen 10, 30, mittels derer die elektrisch leitenden Strukturen 10a, 10b, 10c, 11a, 11b, 11c, 30a, 30b, 30c, 31a, 31b, 31c in den beiden Lagen 10, 30 elektrisch voneinander isoliert werden. Durch diese Maßnahme wird in vorteilhafter Weise die Breite der nicht elektrisch leitenden streifenförmigen Strukturen 12 der ersten Lage 10 und/oder der dritten Lage 30 minimiert, so dass der sensorisch aktive Flächenanteil des Formgestricks 1e entsprechend maximiert werden kann.

Die drei Lagen 10, 20, 30 des Formgestricks 1e können vorzugsweise in einem Strickvorgang so aufeinander gestrickt werden, dass die erste Lage 10 die elektrisch leitenden, streifenförmigen Strukturen 10a, 10b, 10c, 11a, 11b, 11c in der ersten (waagerechten) und die dritte Lage 30 die elektrisch leitenden, streifenförmigen Strukturen 30a, 30b, 30c, 31a, 31b, 31c in der zweiten (senkrechten) Richtung umfasst und die druckabhängig elektrisch leitfähige zweite Lage 20 als Isolierschicht zwischen diesen angeordnet ist. Durch eine im Zeitverlauf alternierende elektrische Ansteuerung der elektrisch leitenden Strukturen 10a, 10b, 10c, 11a, 11b, 11c der ersten Lage 10 und der elektrisch leitenden Strukturen 30a, 30b, 30c, 31a, 31b, 31c der dritten Lage 30 kann durch eine Bestimmung von waagerechten und senkrechten Koordinaten eine ortsaufgelöste Detektion einer Annäherung und/oder Berührung erfolgen. Die Ortsauflösung, die mit der sensorisch aktiven Flächenstruktur erhalten werden kann, hängt insbesondere von der stricktechnischen Flächenaufteilung in leitfähige und nicht leitfähige Flächen beziehungsweise Bereiche ab. Dabei sind die Überschneidungen der Flächen in den beiden Elektrodenebenen entscheidend, die sich beginnend bei einer einzelnen Maschenreihe oder einem Flottfaden in Maschenreihenrichtung respektive einem einzelnen Maschenstäbchen oder einem Stehfaden in Maschenstäbchenrichtung ausbilden.

Ferner können die drei Lagen 10, 20, 30 bei der Herstellung stricktechnisch in definierter Weise so miteinander verbunden werden, dass bestimmte Abstände oder Berührungen eingestellt werden können, beispielsweise als Abstandsgestrick. Ebenfalls sind spezielle Flächenformen stricktechnisch herstellbar, die sowohl 2-dimensional als auch 3-dimensional drapierbar sind.

Es ist auch möglich, die einzelnen Lagen 10, 20, 30 des dreilagigen Formgestricks 1e zunächst einzeln stricktechnisch herzustellen und anschließend konfektionstechnisch, insbesondere durch Vernähen oder Verkleben, miteinander zu verbinden.

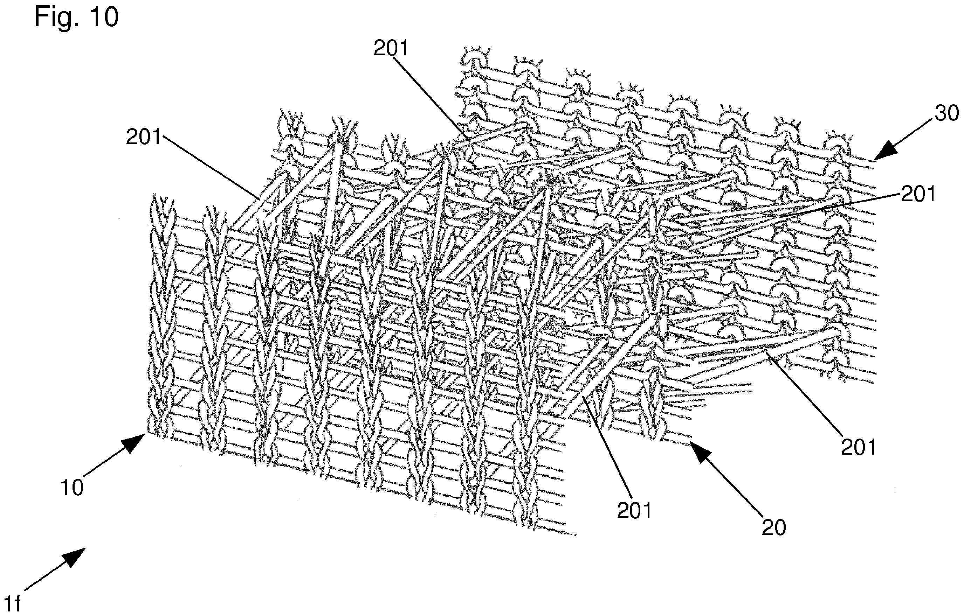

Eine weitere Art der berührungssensitiven Sensorik wird möglich, wenn die zweite Lage 20 nicht als (flächenartige) Zwischenschicht ausgebildet ist, sondern eine Mehrzahl punktueller Abstandshalter 201 zu der ersten Lage 10 und zu der dritten Lage 30 umfasst. Ein derartiges Formgestrick 1f ist in Fig. 10 in einer auseinandergezogenen Ansicht gezeigt. Die elektrisch leitenden Strukturen der ersten Lage 10 und die elektrisch leitenden Strukturen der dritten Lage 30 weisen nach dem Anschluss an eine Spannungsversorgungseinrichtung der Auswerteschaltung 50 ein unterschiedliches elektrisches Potential auf. Bei einer Druckeinwirkung wird der mechanische Widerstand der (insbesondere gestrickten) Fäden, welche die Abstandshalter 201 bilden, überwunden, so dass die erste Lage 10 und die dritte Lage 30 miteinander in Kontakt kommen. Dadurch entsteht ein elektrisches Signal, das von der Auswerteeinheit der Auswerteschaltung 50 wiederum mit Ortsauflösung erfasst werden kann.

Die Herstellung eines solchen mehrlagigen Formgestricks 1f kann insbesondere auf einer zweibettigen Rechts-Rechts-Flachstrickmaschine erfolgen, wobei zum Beispiel auf einem vorderen Nadelbett die erste Lage 10 (auf den vorderen Fadenführerschienen) in einem Intarsienverfahren gestrickt wird und auf einem hinteren Nadelbett mit einem mittleren Fadenführer die zweite Lage 20 auf den geradzahligen Nadeln gestrickt wird und auf den ungeradzahligen Nadeln die dritte Lage 30 mit zwei Fadenführern für leitfähiges und nicht leitfähiges Garn (zum Beispiel auf den beiden hintersten Fadenführerschienen) als Ringelstruktur gestrickt wird.

Die mechanische Verbindung der drei Lagen 10, 20, 30 untereinander soll vorzugsweise nur in denjenigen Bereichen erfolgen, die auf der Elektrodenseite nicht leitfähig sind. Die Verbindung kann zum Beispiel durch Fanghenkel oder durch das Einhängen von Maschen der zweiten (mittleren) Lage in die nichtleitenden Strukturen 12 der ersten und dritten Lage 10, 30 erfolgen beziehungsweise durch Fanghenkel beziehungsweise das Einhängen von Maschen aus den elektrisch nichtleitenden Strukturen 12 der ersten und dritten Lage 10, 30 in die zweite (mittlere) Lage 20.

Nachfolgend sollen weitere Einzelheiten der Herstellung des dreilagigen Formgestricks 1f unter erneuter Bezugnahme auf Fig. 10 näher erläutert werden.

Die erste Lage 10 wird auf einem vorderen Nadelbett mittels jeder zweiten Nadel, wie zum Beispiel den ungeradzahligen Nadeln gestrickt. Die dritte Lage 30 wird auf einem hinteren Nadelbett auch mittels jeder zweiten Nadel, wie zum Beispiel den ungeradzahligen Nadeln, gestrickt. Die zweite (mittlere) Lage 20 wird je nach Bedarf auf den verbleibenden (geradzahligen) Nadeln auf dem vorderen oder dem hinteren Nadelbett gestrickt.

Die drei Lagen werden 10, 20, 30 mit einem weiteren Faden zusammengehalten, der als Fanghenkel in die jeweiligen zusammenzuhängenden Lagen eingelegt wird. Dabei handelt es sich um den Faden mit den geradegestreckten Bereichen in Fig. 10.

Um die erste und zweite Lage 10, 20 zu fertigen, wird die zweite Lage 20 nach hinten umgehängt. Dann werden viermal die erste Lage 10 und zweimal die zweite Lage 20 mit je zwei Verbindungsreihen als Fanghenkel gestrickt. Danach wird die zweite Lage 20 nach vorne gehängt und die zweite und dritte Lage 20, 30 werden entsprechend gebildet. Hierzu werden viermal die dritte Lage 30 gestrickt und zweimal die zweite Lage 20 mit erneut zwei Verbindungsreihen als Fanghenkel gestrickt. Danach beginnt der Ablauf von vorn. Der Abstand der Lagen 10, 20, 30 des Formgestricks 1f kann über den Relativ-Versatz der Nadelbetten zueinander und die Art oder Länge der Verbindungsfäden und der Einbindungshäufigkeit eingestellt werden.

## Patentansprüche

1. Formgestrick (1a-1f), umfassend zumindest eine erste Lage (10), in die mehrere linienartige oder flächenartige, insbesondere streifenförmige, elektrisch leitende Strukturen (10a, 10b, 10c, 11a, 11b, 11c) aus einem elektrisch leitfähigen Garn und linienartige oder flächenartige, insbesondere streifenförmige, elektrisch nichtleitende Strukturen (12) aus einem elektrisch nicht leitfähigen Garn derart eingestrickt sind, dass die elektrisch leitenden Strukturen (10a, 10b, 10c, 11a, 11b, 11c) elektrisch voneinander isoliert sind, wobei jede der elektrisch leitenden Strukturen (10a, 10b, 10c, 11a, 11b, 11c) individuell elektrisch kontaktierbar und an eine Auswerteschaltung (50) anschließbar ist, **dadurch gekennzeichnet, dass** die elektrisch leitenden Strukturen (10a, 10b, 10c, 11a, 11b, 11c) zur elektrischen Kontaktierung punktuell mit isolierten Mikrokabeln oder isolierten leitfähigen Garnen oder Umwindegarnen, die an der Kontaktierungsstelle partiell abisoliert sind, elektrisch verbunden sind, wobei die isolierten Mikrokabel oder isolierten leitfähigen Garne oder Umwindegarne als Stehfäden (20a, 20b, 20c, 21a, 21b, 21c) ausgebildet sind, welche sich parallel zu Maschenstäbchen des Formgestricks (1a-1f) erstrecken.

2. Formgestrick (1a-1f) nach Anspruch 1, **dadurch gekennzeichnet, dass** die elektrisch leitenden Strukturen (10a, 10b, 10c, 11a, 11b, 11c) und/oder die elektrisch nichtleitenden Strukturen (12) der ersten Lage (10) als Intarsiamuster oder eingeringelte Flächen ausgebildet sind.

3. Formgestrick (1a-1f) nach einem der Ansprüche 1 oder 2, **dadurch gekennzeichnet, dass** das Formgestrick (1a-1f) zumindest eine zweite Lage (20) aufweist, die mit der ersten Lage (10) verbunden ist.

4. Formgestrick (1a-1f) nach Anspruch 3, **dadurch gekennzeichnet, dass** die zweite Lage (20) zumindest abschnittsweise eine gestrickte Lage ist, die insbesondere stricktechnisch mit der ersten Lage (10) verbunden ist oder mit der ersten Lage (10) vernäht ist.

5. Formgestrick (1a-1f) nach einem der Ansprüche 3 oder 4, **dadurch gekennzeichnet, dass** die zweite Lage (20) zumindest abschnittsweise ein Gewebe und/oder ein Gewirk und/oder ein Gelege und/oder ein Vliesmaterial und/oder ein Schaummaterial und/oder eine Folie umfasst.

6. Formgestrick (1a-1f) nach einem der Ansprüche 3 bis 5, **dadurch gekennzeichnet, dass** die zweite Lage (20) zumindest abschnittsweise aus einem elektrisch nichtleitenden Werkstoff hergestellt ist.

7. Formgestrick (1a-1f) nach einem der Ansprüche 3 bis 6, **dadurch gekennzeichnet, dass** die zweite Lage (20) zumindest abschnittsweise aus einem elastisch verformbaren Werkstoff hergestellt ist.

8. Formgestrick (1a-1f) nach einem der Ansprüche 3 bis 7, **dadurch gekennzeichnet, dass** das Formgestrick (1a-1f) eine dritte Lage (30) aufweist, in die mehrere linienartige oder flächenartige, insbesondere streifenförmige, elektrisch leitende Strukturen (30a, 30b, 30c, 31a, 31b, 31c) aus einem elektrisch leitfähigen Garn, vorzugsweise als Intarsiamuster oder eingeringelte Flächen, und linienartige oder flächenartige, insbesondere streifenförmige, elektrisch nichtleitende Strukturen (12) aus einem elektrisch nicht leitfähigen Garn, vorzugsweise als Intarsiamuster oder eingeringelte Flächen, derart eingestrickt sind, dass die elektrisch leitenden Strukturen (30a, 30b, 30c, 31a, 31b, 31c) elektrisch voneinander isoliert sind, wobei jede der elektrisch leitenden Strukturen (30a, 30b, 30c, 31a, 31b, 31c) individuell elektrisch kontaktierbar und an eine Auswerteschaltung (50) anschließbar ist.

9. Formgestrick (1a-1f) nach Anspruch 8, **dadurch gekennzeichnet, dass** sich die linienartigen oder streifenförmigen elektrisch leitenden Strukturen (10a, 10b, 10c, 11a, 11b, 11c) der ersten Lage (10) parallel zueinander in einer ersten Richtung erstrecken und dass sich die linienartigen oder streifenförmigen elektrisch leitenden Strukturen (30a, 30b, 30c, 31a, 31b, 31c) der dritten Lage (30) parallel zueinander in einer zweiten Richtung erstrecken, die von der ersten Richtung verschieden ist.

10. Formgestrick (1a-1f) nach einem der Ansprüche 8 oder 9, **dadurch gekennzeichnet, dass** die Breite der streifenförmigen elektrisch leitenden Strukturen (10a, 10b, 10c, 11a, 11b, 11c) der ersten Lage (10) und/oder die Breite der streifenförmigen elektrisch leitenden Strukturen (30a, 30b, 30c, 31a, 31b, 31c) der dritten Lage (30) größer als die Breite der benachbarten, nichtleitenden Strukturen (12) der betreffenden Lage (10, 30) ist.

11. Formgestrick (1a-1f) nach einem der Ansprüche 8 bis 10, **dadurch gekennzeichnet, dass** die zweite Lage (20) eine Mehrzahl punktueller Abstandshalter (201) zu der ersten Lage (10) und/oder zu der dritten Lage (30) umfasst.

12. Verwendung eines Formgestricks (1a-1f) nach einem der Ansprüche 1 bis 11 und einer daran angeschlossenen Auswerteschaltung (50) als Sensoranordnung.

13. Verwendung eines Formgestricks (1a-1f) nach einem der Ansprüche 1 bis 11 als Schaltvorrichtung und/oder als Eingabevorrichtung.

## Claims

1. Knitted fabric (1a-1f) comprising at least one first layer (10) into which a plurality of linear or flat, in particular strip-shaped, electroconductive structures (10a, 10b, 10c, 11a, 11b, 11c) made of an electroconductive yarn and linear or flat, in particular strip-shaped, non-electroconductive structures (12) made of a non-electroconductive yarn are knitted in such way that the electroconductive structures (10a, 10b, 10c, 11a, 11b, 11c) are electrically insulated from one another, wherein each of the electroconductive structures (10a, 10b, 10c, 11a, 11b, 11c) is capable of being individually electrically contacted and connected to an evaluation circuit (50), **characterised in that**, in order to make electrical contact, the electroconductive structures (10a, 10b, 10c, 11a, 11b, 11c) are electrically connected in a punctiform manner with insulated microcables or insulated conductive yarns or wrapped yarns which are partially stripped at the contact point, wherein the insulated microcables or insulated conductive yarns or wrapped yarns are configured as filler threads (20a, 20b, 20c, 21a, 21b, 21c) which extend parallel to stitch wales of the knitted fabric (1a-1f).

2. Knitted fabric (1a-1f) according to claim 1, **characterised in that** the electroconductive structures (10a, 10b, 10c, 11a, 11b, 11c) and/or the non-electroconductive structures (12) of the first layer (10) are configured as Intarsia patterns or encircled areas.

3. Knitted fabric (1a-1f) according to any of claims 1 or 2, **characterised in that** the knitted fabric (1a-1f) has at least one second layer (20) which is connected to the first layer (10).

4. Knitted fabric (1a-1f) according to claim 3, **characterised in that** the second layer (20) is, at least in sections, a knitted layer which is in particular connected to the first layer (10) by knitting or is sewn to the first layer (10).

5. Knitted fabric (1a-1f) according to any of claims 3 or 4, **characterised in that** the second layer (20) comprises, at least in sections, a woven fabric and/or a warp knitted fabric and/or a scrim and/or a non-woven material and/or a foam material and/or a film.

6. Knitted fabric (1a-1f) according to any of claims 3 to 5, **characterised in that** the second layer (20) is, at least in sections, made of a non-electroconductive material.

7. Knitted fabric (1a-1f) according to any of claims 3 to 6, **characterised in that** the second layer (20) is, at least in sections, made of an elastically deformable material.

8. Knitted fabric (1a-1f) according to any of claims 3 to 7, **characterised in that** the knitted fabric (1a-1f) has a third layer (30) into which a plurality of linear or flat, in particular strip-shaped, electroconductive structures (30a, 30b, 30c, 31a, 31b, 31c) made of an electroconductive yarn, preferably as Intarsia patterns or encircled areas, and linear or flat, in particular strip-shaped, non-electroconductive structures (12) made of a non-electroconductive yarn, preferably as Intarsia patterns or encircled areas, are knitted such that the electroconductive structures (30a, 30b, 30c, 31a, 31b, 31c) are electrically insulated from one another, wherein each of the electroconductive structures (30a, 30b, 30c, 31a, 31b, 31c) are capable of being individually electrically contacted and connected to an evaluation circuit (50).

9. Knitted fabric (1a-1f) according to claim 8, **characterised in that** the linear or strip-shaped electroconductive structures (10a, 10b, 10c, 11a, 11b, 11c) of the first layer (10) extend parallel to one another in a first direction and that the linear or strip-shaped electroconductive structures (30a, 30b, 30c, 31a, 31b, 31c) of the third layer (30) extend parallel to one another in a second direction which is different from the first direction.

10. Knitted fabric (1a-1f) according to any of claims 8 or 9, **characterised in that** the width of the strip-shaped electroconductive structures (10a, 10b, 10c, 11a, 11b, 11c) of the first layer (10) and/or the width of the strip-shaped electroconductive structures (30a, 30b, 30c, 31a, 31b, 31c) of the third layer (30) is greater than the width of the adjacent non-conductive structures (12) of the respective layer (10, 30).

11. Knitted fabric (1a-1f) according to any of claims 8 to 10, **characterised in that** the second layer (20) comprises a plurality of punctiform spacers (201) from the first layer (10) and/or from the third layer (30).

12. Use of a knitted fabric (1a-1f) according to any of claims 1 to 11 and an evaluation circuit (50) connected thereto as a sensor arrangement.

13. Use of a knitted fabric (1a-1f) according to any of claims 1 to 11 as a switching device and/or as an input device.

## Revendications

1. Tricotage (la-lf), comprenant au moins une première couche (10), dans laquelle plusieurs structures électroconductrices (10a, 10b, 10c, 11a, 11b, 11c) en forme de lignes ou de surfaces, en particulier en forme de bandes, sont tricotées en un fil électroconducteur et des structures non électroconductrices (12) en forme de lignes ou de surfaces, en particulier en forme de bandes, sont tricotées en un fil non électroconducteur, de sorte que les structures électroconductrices (10a, 10b, 10c, 11a, 11b, 11c) sont électriquement isolées les unes des autres, dans lequel chacune des structures électroconductrices (10a, 10b, 10c, 11a, 11b, 11c) peut être mise en contact électrique individuellement et raccordées à un circuit d'évaluation (50), **caractérisé en ce que** les structures électroconductrices (10a, 10b, 10c, 11a, 11b, 11c) sont connectées ponctuellement, pour la mise en contact électrique, à des microcâbles isolés ou à des fils conducteurs isolés ou à des fils de guipage, qui sont partiellement dénudés au niveau du point de contact, dans lequel les microcâbles isolés ou les fils conducteurs isolés ou les fils de guipage sont conçus en tant que fils fixes (20a, 20b, 20c, 21a, 21b, 21c), lesquels s'étendent parallèlement aux colonnes de mailles du tricotage (1a-1f).

2. Tricotage (1a-1f) selon la revendication 1, **caractérisé en ce que** les structures électroconductrices (10a, 10b, 10c, 11a, 11b, 11c) et/ou les structures non électroconductrices (12) de la première couche (10) sont conçues en tant que motifs intarsia ou surfaces bouclées.

3. Tricotage (1a-1f) selon l'une quelconque des revendications 1 ou 2, **caractérisé en ce que** le tricotage (1a-1f) présente au moins une deuxième couche (20), qui est connectée à la première couche (10).

4. Tricotage (1a-1f) selon la revendication 3, **caractérisé en ce que** la deuxième couche (20) est, au moins par sections, une couche tricotée, qui est en particulier connectée à la première couche (10) par une technique de tricotage ou cousue à la première couche (10).

5. Tricotage (1a-1f) selon l'une quelconque des revendications 3 ou 4, **caractérisé en ce que** la deuxième couche (20) comprend au moins par sections un tissu tissé et/ou un tissu à mailles et/ou un canevas et/ou un matériau non tissé et/ou un matériau en mousse et/ou un film.

6. Tricotage (1a-1f) selon l'une quelconque des revendications 3 à 5, **caractérisé en ce que** la deuxième couche (20) est fabriquée au moins par sections en un matériau non électroconducteur.

7. Tricotage (1a-1f) selon l'une quelconque des revendications 3 à 6, **caractérisé en ce que** la deuxième couche (20) est fabriquée au moins par sections en un matériau élastiquement déformable.

8. Tricotage (1a-1f) selon l'une quelconque des revendications 3 à 7, **caractérisé en ce que** le tricotage (1a-1f) présente une troisième couche (30), dans laquelle plusieurs structures électroconductrices (30a, 30b, 30c, 31a, 31b, 31c) en forme de lignes ou de surfaces, de préférence en forme de bandes, sont tricotées en un fil électroconducteur, de préférence en tant que motif intarsia ou surfaces bouclées, et des structures non électroconductrices (12) en forme de lignes ou de surfaces, en particulier en forme de bandes, sont tricotées en un fil non électroconducteur, de préférence en tant que motif intarsia ou surfaces bouclées, de sorte que les structures électroconductrices (30a, 30b, 30c, 31a, 31b, 31c) sont isolées électriquement les unes des autres, dans lequel chacune des structures électroconductrices (30a, 30b, 30c, 31a, 31b, 31c) peut être mise en contact électrique individuellement et raccordée à un circuit d'évaluation (50).

9. Tricotage (1a-1f) selon la revendication 8, **caractérisé en ce que** les structures électroconductrices en forme de lignes ou en forme de bandes (10a, 10b, 10c, 11a, 11b, 11c) de la première couche (10) s'étendent parallèlement les unes aux autres dans une première direction et **en ce que** les structures électroconductrices en forme de lignes ou en forme de bandes (30a, 30b, 30c, 31a, 31b, 31c) de la troisième couche (30) s'étendent parallèlement les unes aux autres dans une deuxième direction, qui est différente de la première direction.

10. Tricotage (1a-1f) selon l'une quelconque des revendications 8 ou 9, **caractérisé en ce que** la largeur des structures électroconductrices en forme de bandes (10a, 10b, 10c, 11a, 11b, 11c) de la première couche (10) et/ou la largeur des structures électroconductrices en forme de bandes (30a, 30b, 30c, 31a, 31b, 31c) de la troisième couche (30) est supérieure à la largeur des structures non conductrices voisines (12) de la couche concernée (10, 30).

11. Tricotage (1a-1f) selon l'une quelconque des revendications 8 à 10, **caractérisé en ce que** la deuxième couche (20) comprend une pluralité d'espaceurs ponctuels (201) par rapport à la première couche (10) et/ou à la troisième couche (30).

12. Utilisation d'un tricotage (1a-1f) selon l'une quelconque des revendications 1 à 11 et d'un circuit d'évaluation (50) qui lui est raccordé en tant que dispositif de détection.

13. Utilisation d'un tricotage (1a-1f) selon l'une quelconque des revendications 1 à 11 en tant que dispositif de commutation et/ou en tant que dispositif d'entrée.
